# FASCICULE DE BREVET EUROPEEN

(11) **EP 3 077 354 B1**
(45) Date de publication et mention de la délivrance du brevet: **29.11.2017**
(21) Numéro de dépôt: 14821794.6
(22) Date de dépôt: 04.12.2014
(51) Int. Cl.: C07C 41/16, C07C 41/18, C07C 41/26, C07C 41/32, C07C 303/28, C07C 303/30, C07C 43/23, C07C 309/65, C07C 309/66, C07C 309/73, C07C 45/30, C07C 213/02, C07C 217/58, C07C 217/60, C07C 231/02

(54) **NOUVEAU PROCEDE DE SYNTHESE DE L'AGOMELATINE**
NEUARTIGES VERFAHREN ZUR SYNTHESE VON AGOMELATIN
NOVEL METHOD FOR THE SYNTHESIS OF AGOMELATINE

(30) Priorité: 05.12.2013 FR 1362198
(43) Date de publication de la demande: 12.10.2016
(73) Titulaire: Les Laboratoires Servier, 92284 Suresnes (FR)
(72) Inventeur: BRIERE, Jean-François, F-76920 Amfreville-la-Mi-Voie (FR); LEBEUF, Raphaël, F-59000 Lille (FR); LEVACHER, Vincent, F-76160 Fontaine-sous-Preaux (FR); HARDOUIN, Christophe, 76310 Sainte Adresse (FR); LECOUVE, Jean-Pierre, F-76600 Le Havre (FR)
(86) Numéro de dépôt international: PCT/FR2014/053157
(87) Numéro de publication internationale: WO 2015/082847

(56) Documents cités:
- EP-A1- 0 447 285
- EP-A1- 1 564 202
- EP-A1- 2 151 427
- EP-A1- 2 322 508
- LI P-K ET AL: "The development of a charged melatonin receptor ligand", BIOORGANIC & MEDICINAL CHEMISTRY LETTERS, PERGAMON, AMSTERDAM, NL, vol. 7, no. 18, 23 septembre 1997 (1997-09-23), pages 2409-2414, XP004136454, ISSN: 0960-894X, DOI: 10.1016/S0960-894X(97)00444-7

## Description

La présente invention concerne un nouveau procédé de synthèse industriel de l'agomélatine ou *N*-[2-(7-méthoxy-1-naphtyl)éthyl]acétamide de formule (I) :

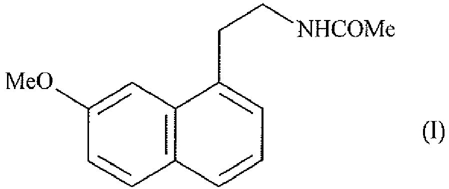

L'agomélatine ou *N*-(2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans les brevets européens EP 0 447 285 et EP 1 564 202.

Compte-tenu de l'intérêt pharmaceutique de ce composé, il était important de pouvoir y accéder avec un procédé de synthèse industriel performant, facilement transposable à l'échelle industrielle, conduisant à l'agomélatine avec un bon rendement, et une excellente pureté.

Le brevet EP 0 447 285 décrit l'accès en huit étapes à l'agomélatine à partir de la 7-méthoxy-1-tétralone. Dans le brevet EP 1 564 202, la demanderesse a mis au point une nouvelle voie de synthèse beaucoup plus performante et industrialisable, en seulement quatre étapes à partir de la 7-méthoxy-1-tétralone, et permettant d'obtenir l'agomélatine de façon très reproductible sous une forme cristalline bien définie. Toutefois, la recherche de nouvelles voies de synthèse, en particulier à partir de matières premières moins onéreuses que la 7-méthoxy-1-tétralone, est toujours d'actualité.

La demanderesse a poursuivi ses investigations et a mis au point un nouveau procédé de synthèse de l'agomélatine à partir du 7-méthoxy-naphtalén-2-ol : cette nouvelle matière première présente l'avantage d'être simple, aisément accessible en grandes quantités avec des coûts moindres. Le 7-méthoxy-naphtalén-2-ol présente également l'avantage de posséder dans sa structure un noyau naphtalène, ce qui évite d'intégrer dans la synthèse une étape d'aromatisation, étape toujours délicate d'un point de vue industriel.
Ce nouveau procédé permet par ailleurs d'obtenir l'agomélatine de façon reproductible et sans nécessiter de purification laborieuse, avec une pureté qui est compatible avec son utilisation comme principe actif pharmaceutique.

Plus spécifiquement, la présente invention concerne un procédé de synthèse industriel du composé de formule (I) : 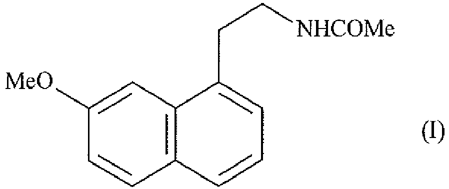 caractérisé en ce que l'on met en réaction le 7-méthoxy-naphtalén-2-ol de formule (II) : 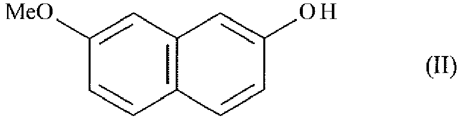 sur lequel on introduit sur la position 1 du composé de formule (II) le groupement -CH₂-X dans lequel X représente un groupement -N(CH₃)₂, -CO-N(CH₂-Ph)₂, -CH₂-OH, -CH=CH₂ ou -CO-NH₂,
pour conduire au composé de formule (III) : 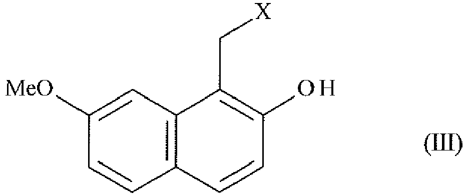 dans laquelle X représente un groupement -N(CH₃)₂, -CO-N(CH₂-Ph)₂, -CH₂-OH, -CH=CH₂ ou -CO-NH₂ ;
composé de formule (III) qui est soumis à une réaction de sulfonylation sur l'alcool aromatique et dont le substituant X est modifié, avant ou après l'étape de sulfonylation de l'alcool aromatique, au moyen de réactions chimiques classiques pour conduire au composé de formule (IV) : 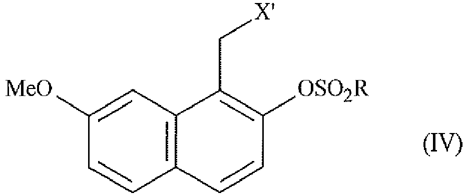 dans laquelle X' représente un groupement -CN, -CO-NH₂, -CH₂-OH, -CH₂-N(CH₂-Ph)₂, -CH₂-NH-CO-CH₃, -CH(OH)-CH₂-OH, -CHO ou (2,5-dioxopyrrolidin-1-yl)méthyle et R représente un groupement -CH₃, -(CH₂)₂-CH₃, -CF₃ ou toluyle ;
composé de formule (IV) qui subit une réaction de désoxygénation en présence d'un métal de transition et d'un agent réducteur pour conduire :
- soit, lorsque X' représente le groupement -CH₂-NH-CO-CH₃, directement au composé de formule (I) que l'on isole sous forme d'un solide ;
- soit au composé de formule (V) : 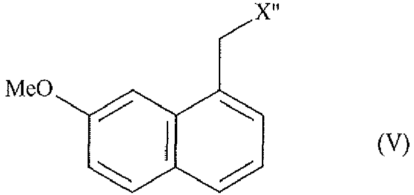
dans laquelle X" représente un groupement -CN, -CH₂-N(CH₂-Ph)₂, -CH₂-OH, -CO-NH₂, -CH(OH)-CH₂-OH ou (2,5-dioxopyrrolidin-1-yl)méthyle ;
composé de formule (V) qui est ensuite soumis à des réactions classiques de chimie pour conduire au composé de formule (I) que l'on isole sous forme d'un solide.

Une variante au procédé de synthèse industriel consiste en ce que, lors de la transformation du composé de formule (III) en composé de formule (IV), le groupement X n'est pas modifié. Le composé obtenu sulfonylé sur son alcool aromatique subit alors une réaction de désoxygénation par action d'un métal de transition et d'un agent réducteur. Le groupement X est modifié ultérieurement grâce à des réactions classiques de chimie pour conduire au composé de formule (I) que l'on isole sous forme d'un solide.

Le composé de formule (II) est commercial ou aisément accessible à l'homme du métier par des réactions de chimie classiques ou décrites dans la littérature.

Dans le procédé selon l'invention, la transformation du composé de formule (II) en composé de formule (III) pour lequel X représente le groupement -N(CH₃)₂, est réalisée selon la réaction de Mannich par action du formaldéhyde en présence de diméthylamine.

Dans le procédé selon l'invention, la transformation du composé de formule (II) en composé de formule (III) pour lequel X représente le groupement -CH₂-OH, consiste en ce que le composé de formule (II) est soumis à l'action du glyoxal (ou éthane-1,2-dione) suivie de l'action d'un agent réducteur. De manière avantageuse, l'agent réducteur est l'hydrure de lithium et d'aluminium, l'hydrure de diisobutylaluminium, le triéthylborohydrure de lithium ou le borane-diméthylsulfure. Préférentiellement, l'agent réducteur est l'hydrure de lithium et d'aluminium.

Dans le procédé selon l'invention, la transformation du composé de formule (II) en composé de formule (III) pour lequel X représente le groupement -CO-NH₂ ou -CO-N(CH₂-Ph)₂, est réalisée par action du glyoxal suivie de l'action en milieu chauffé du composé de formule NHR'R' dans laquelle R' représente H ou un groupement -CH₂-Ph.

Ladite réaction avec le glyoxal qui conduit à la formation de la lactone intermédiaire de formule (VI) : 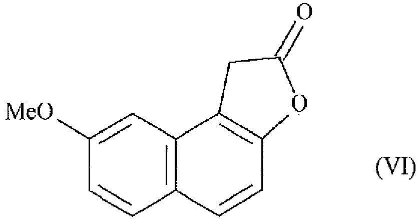 est préférentiellement réalisée en deux étapes.
Dans la première étape, le composé de formule (II) est mis en solution dans un milieu basique en présence du glyoxal. La base est, de préférence, l'hydroxyde de sodium ou l'hydroxyde de potassium et, plus particulièrement, l'hydroxyde de potassium.
Dans la deuxième étape, le produit intermédiaire, à savoir le 8-méthoxy-1,2-dihydronaphto(2,1-*b*]furane-1,2-diol, est directement mis en solution en milieu acide, de préférence l'acide chlorhydrique, pour conduire à la lactone intermédiaire de formule (VI).

Dans le procédé selon l'invention, la transformation du composé de formule (II) en composé de formule (III) pour lequel X représente le groupement -CH=CH₂, est réalisée selon le réarrangement sigmatropique de Claisen par action du bromure d'allyle en milieu basique suivie d'un réarrangement thermique. L'action du bromure d'allyle se fait en présence d'une base telle que l'hydrure de sodium, le *tert*-butylate de potassium, le méthanolate de sodium, l'hydroxyde de potassium, l'hydroxyde de sodium, le carbonate de potassium ou le carbonate de sodium. Un mode de réalisation avantageux consiste à utiliser le carbonate de potassium comme base lors de l'étape de réaction avec le bromure d'allyle.

Dans le procédé selon l'invention, la transformation du composé de formule (III) en composé de formule (IV) consiste en une étape de sulfonylation de l'alcool aromatique suivie par la modification du groupement X au moyen de réactions classiques de chimie, X étant défini tel que précédemment. Selon un autre mode de réalisation avantageux, la transformation du composé de formule (III) en composé de formule (IV) consiste en la modification du groupement X au moyen de réactions classiques de chimie suivie par une étape de sulfonylation de l'alcool aromatique, X étant défini tel que précédemment.

Ladite étape de sulfonylation est réalisée grâce à l'action d'un chlorure de sulfonyle, d'un anhydride sulfonique ou d'un sulfonimide. Selon un mode de réalisation préféré, l'étape de sulfonylation est réalisée grâce à l'action du chlorure de tosyle, du chlorure de *n*-propylsulfonyle, de l'anhydride triflique ou du phényltriflimide (ou *N,N*-bis(trifluorométhylsulfonyl)aniline).

Dans le procédé selon l'invention, la transformation du composé de formule (IV) en composé de formule (V) consiste en une étape de désoxygénation en présence d'un métal de transition et d'un agent réducteur.
De manière préférée, le métal de transition est le nickel, le palladium ou le platine. Le métal de transition peut être soit sous forme d'un sel ou soit sous forme d'un corps simple. Préférentiellement, le sel de métal de transition est un sel de nickel ou un sel de palladium, plus préférentiellement un sel de nickel.
Avantageusement, l'agent réducteur est soit un hydrure tel que le borohydrure de sodium ou l'hydrure de lithium et d'aluminium ; soit un aminoborane tel que le diméthylamine-borane ; soit un alkoxysilane tel que le diméthoxyméthylsilane ; soit un alkylsilane tel que le triéthylsilane ; soit un métal alcalino-terreux tel que le magnésium ; soit le dihydrogène. Selon un autre mode de réalisation préféré, la transformation du composé de formule (IV) en composé de formule (V) consiste en une étape de désoxygénation en présence de nickel, en particulier un sel de nickel, et d'un hydrure, préférentiellement le borohydrure de sodium.
Selon un autre mode de réalisation préféré, la transformation du composé de formule (IV) en composé de formule (V) consiste en une étape de désoxygénation en présence de palladium et de dihydrogène. Le dihydrogène est utilisé directement sous sa forme gazeuse ou bien est indirectement obtenu par décomposition d'un formiate d'ammonium.
Selon un autre mode de réalisation préféré, la transformation du composé de formule (IV) en composé de formule (V) consiste en une étape de désoxygénation en présence de palladium et d'un métal alcalino-terreux, préférentiellement, le magnésium.
Selon un autre mode de réalisation préféré, la transformation du composé de formule (IV) en composé de formule (V) consiste en une étape de désoxygénation en présence d'un métal de transition, d'un agent réducteur et d'un ligand. Le ligand est préférentiellement un ligand phosphine et, plus particulièrement, la triphénylphosphine.

Selon un mode de réalisation particulier, l'étape de désoxygénation du composé de formule (IV) dans laquelle X' représente le groupement -CH₂-NH-CO-CH₃, effectuée :
- soit en présence de nickel, en particulier un sel de nickel, et d'un hydrure, préférentiellement le borohydrure de sodium ;
- soit en présence de palladium et de dihydrogène ;
- soit en présence de palladium et d'un métal alcalino-terreux ;
conduit directement à la formation du composé de formule (I).

Ce procédé est particulièrement intéressant pour les raisons suivantes :
- il permet d'obtenir à l'échelle industrielle le composé de formule (I) avec de bons rendements à partir d'une matière première simple et peu onéreuse ;
- il permet d'éviter une réaction d'aromatisation, étape toujours délicate d'un point de vue industriel, puisque le noyau naphtalénique est présent dans le substrat de départ ;
- il permet d'obtenir l'agomélatine à partir du 7-méthoxy-naphtalén-2-ol avec un nombre réduit d'étapes.

Les composés de formule (III), (IV) et (VI) obtenus selon le procédé de l'invention sont nouveaux et utiles en tant qu'intermédiaire de synthèse de l'agomélatine.

Les composés de formule (V) obtenus selon le procédé de l'invention sont utiles en tant qu'intermédiaire de synthèse de l'agomélatine. Les composés de formule (V) obtenus selon le procédé de l'invention sont nouveaux, exceptés le (7-méthoxynaphtalén-1-yl) acétonitrile, le *N,N*-dibenzyl-2-(7-méthoxynaphtalén-1-yl)éthanamine, le 2-(7-méthoxy naphtalén-1-yl)éthanol et le 2-(7-méthoxynaphtalén-1-yl)acétamide.

Les composés de formule (III) préférés sont les suivants :
- 1-[(diméthylamino)méthyl]-7-méthoxynaphtalén-2-ol ;
- *N,N*-dibenzyl-2-(2-hydroxy-7-méthoxynaphtalén-1-yl)acétamide ;
- 1-(2-hydroxyéthyl)-7-méthoxynaphtalén-2-ol ;
- 2-(2-hydroxy-7-méthoxynaphtalén-1-yl)acétamide ;
- 7-méthoxy-1-(prop-2-én-1-yl)naphtalén-2-ol.

Les composés de formule (IV) préférés sont les suivants :
- trifluorométhanesulfonate de 1-(cyanométhyl)-7-méthoxynaphtalén-2-yle ;
- 4-méthylbenzènesulfonate de 1-[2-(acétylamino)éthyl]-7-méthoxynaphtalén-2-yle ;
- trifluorométhanesulfonate de 1-[2-(dibenzylamino)éthyl]-7-méthoxynaphtalén-2-yle;
- propane-1-sulfonate de 1-[2-(acétylamino)éthyl]-7-méthoxynaphtalén-2-yle ;
- propane-1-sulfonate de 1-[2-(2,5-dioxopyrrolidin-1-yl)éthyl]-7-méthoxynaphtalén-2-yle ;
- 4-méthylbenzènesulfonate de 1-(2-hydroxyéthyl)-7-méthoxynaphtalén-2-yle ;
- 4-méthylbenzènesulfonate de 1-(2-amino-2-oxoéthyl)-7-méthoxynaphtalén-2-yle ;
- 4-méthylbenzènesulfonate de 7-méthoxy-1-(2-oxoéthyl)naphtalén-2-yle ;
- 4-méthylbenzènesulfonate de 1-(2,3-dihydroxypropyl)-7-méthoxynaphtalén-2-yle.

Le (2-hydroxy-7-méthoxynaphtalén-1-yl)acétonitrile, le 4-méthylbenzènesulfonate de 7-méthoxy-1-(2-{[(4-méthylphényl)sulfonyl]oxy}éthyl)naphtalén-2-yle, le propane-1-sulfonate de 7-méthoxy-1-{2-[(propylsulfonyl)oxy]éthyl}naphtalén-2-yle et l'acétate de 1-[2-(acétylamino)éthyl]-7-méthoxynaphtalén-2-yle sont nouveaux et utiles en tant qu'intermédiaire de synthèse de l'agomélatine.

Les exemples ci-dessous illustrent l'invention, mais ne la limitent en aucune façon.
Afin de bien valider les chemins réactionnels, les intermédiaires de synthèse ont été systématiquement isolés et caractérisés. Toutefois, il est possible d'optimiser considérablement les procédés en limitant le nombre d'intermédiaires isolés.
Les structures des composés décrits ont été confirmées par les techniques spectroscopiques usuelles : RMN du proton (s = singulet ; ls = large singulet ; d = doublet ; t = triplet ; dd = doublet dédoublé ; m = multiplet) ; RMN du carbone (s = singulet ; d = doublet ; t = triplet ; q = quadruplet) ; spectrométrie de masse par ionisation par électrospray (ESI).

### EXEMPLE 1 : N-[2-(7-méthoxynaphtalén-1-yl)éthyl]acétamide

### Stade A : 1-[(diméthylamino)méthyl]-7-méthoxynaphtalén-2-ol

A une solution de 7-méthoxy-naphtalén-2-ol (1,74 g ; 10 mmol) dans l'éthanol (10 mL), sont ajoutés à température ambiante la diméthylamine (40 % dans l'eau; 1,52 mL ; 12 mmol) puis le formaldéhyde (37 % dans l'eau ; 0,78 mL ; 10,5 mmol). Après une heure d'agitation, le solvant est évaporé. Le brut obtenu (rendement quantitatif) est utilisé directement dans l'étape suivante sans plus de purification.
*Analyse spectroscopique de RMN ¹H (DMSO-d6, 300,13 Mhz, δ en ppm) : 11,18 (ls, 1H) ; 7,68 (d, J = 8,8 Hz, 1H) ; 7,6 (d, J = 8,8 Hz, 1H) ; 7,23 (d, J* = *2,3 Hz, 1H) ; 6,93 (dd, J* = *8,8 et 2,3 Hz, 1H) ; 6,89 (d, J* = *8,8 Hz, 1H) ; 3,96 (s, 2H) ; 3,86 (s, 3H) ; 2,3 (s, 6H).*
*Analyse spectroscopique de RMN ¹³C (DMSO-d6, 75,5 MHz, δ en ppm) : 157,7 (s) ; 156,1 (s)* ; *134,3 (s)* ; *129,9 (d) ; 128,5 (d) ; 123,3 (s) ; 116,0 (d) ; 114,2 (d)* ; *112,0 (s)* ; *101,6 (d) ; 55,8 (t)* ; *55,0 (q) ; 44,3* (2 *x q).*

### Stade B : (2-hydroxy-7-méthoxynaphtalén-1-yl)acétonitrile

Une solution du produit du Stade A précédent (1,155 g; 5 mmol) dans le diméthylformamide (5 mL) en présence de cyanure de potassium (390 mg ; 6 mmol) est chauffée à 80 °C pendant 30 heures. Après dilution dans l'acétate d'éthyle, une solution aqueuse de HCl 2 M (5 mL) est ajoutée. Le mélange est agité puis neutralisé *via* l'ajout d'une solution diluée de NaHCO₃. Les deux phases sont séparées et la fraction organique est lavée trois fois avec de la saumure, séchée sur sulfate de sodium et filtrée. L'évaporation du solvant donne un brut qui est ensuite purifié par chromatographie sur colonne de gel de silice (éluant : éther / éther de pétrole 40/60) pour conduire au produit attendu.
*Analyse spectroscopique de RMN ¹H (acétone-d₆, 300,13 MHz, δ en pppm) : 9,25 (ls, 1H, OH) ; 7,74 (d, J* = *9,1 Hz, 1H) ; 7,71 (d, J = 8,9 Hz, 1H) ; 7,31 (d, J* = *2,5 Hz, 1H) ; 7,13 (d, J* = *8,9 Hz, 1H) ; 7,03 (dd, J* = *9,1 et 2,5 Hz, 1H) ; 4,21 (s, 2H) ; 3,96 (s, 3H).*
*Analyse spectroscopique de RMN ¹³C (acétone-d₆, 75,5 MHz, δ en ppm) : 159,9 (s); 154,1 (s) ; 135,1 (s) ; 131,1 (d) ; 130,4 (d) ; 124,9 (s) ; 119,1 (s) ; 116,2 (d) ; 115,7 (d) ; 108,7 (s) ; 102,1 (d) ; 55,6 (q) ; 13,6 (t).*

### Stade C : trifluorométhanesulfonate de 1-(cycmométhyl)-7-méthoxynaphtalén-2-yle

A une solution du produit du Stade B précédent (120 mg ; 0,52 mmol) dans le dichlorométhane (5 mL), sont ajoutés le *N,N*-bis(trifluorométhylsulfonyl)aniline (204 mg ; 0,571 mmol) et la triéthylamine (72 µL ; 0,52 mmol). Après agitation pendant 16 heures, les solvants sont évaporés et le résidu est purifié par chromatographie sur colonne de gel de silice (éluant : acétate d'éthyle / éther de pétrole, gradient de 10 / 90 à 20 / 80) pour donner le produit du titre (125 mg ; 70 %).
*Analyse spectroscopique de RMN ¹**H* (*CDCl₃**, 300*, *13 MHz, δ en ppm) : 7,85-7,78 (m, 2H)* ; *7,3-7,22 (m, 3H) ; 4,13 (s, 2H) ; 3,98 (s, 3H).*
*Analyse spectroscopique de RMN ¹³C (CDCl₃, 75,5 MHz, δ en ppm) : 159,9 (s) ; 145,6 (s)* ; *133,2 (s)* ; *131,2 (d)* ; *130,8 (d) ; 128,0 (s) ; 125,0 (s) ; 120,3 (d) ; 118,6 (s, J* - *318 Hz)* ; *116,9 (s)* ; *116,8 (d)* ; *116,0 (s)* ; *102,3 (d) ; 55,6 (q) ; 15,2 (t).*
*Spectrométrie de masse (ESI ; m*/*z(%)) : 345(45) [M]*^{+•} *; 212(100) ; 184(15); 169(34) ; 140(18).*

### Stade D : (7-méthoxynaphtalén-1-yl)acétonitrile

Une solution du produit du Stade C précédent (73 mg ; 0,212 mmol) dans l'éthanol absolu (4mL) en présence de palladium à 10% sur charbon (4,5 mg ; 0,004 mmol) et de triéthylamine (150 µL) est hydrogénée (4 bars) à température ambiante pendant 20 heures. Après filtration sur Célite, lavage à l'acétate d'éthyle et évaporation des solvants, le brut est purifié par chromatographié sur colonne de gel de silice (éluant : acétate d'éthyle / éther de pétrole 15 / 85) pour donner le produit du titre (28 mg ; 67 %).
*Point de fusion : 86-87 °C*.
*Analyse spectroscopique de RMN ¹H (CDCl₃*, *300,13 MHz, δ en ppm)* : *7,78 (d, J* = *8,9 Hz, 1H) ; 7,77 (d, J* = *7,8 Hz, 1H) ; 7,52 (d, J* = *7,1 Hz, 1H) ; 7,32 (dd, J* = *7,8 et 7,1 Hz, 1H) ; 7,21 (dd, J* = *8,9 et 2,4 Hz, 1H) ; 7,03 (d, J = 2,4 Hz, 1H) ; 4,0 (s, 2H)* ; *3,94 (s, 3H).*
*Analyse spectroscopique de RMN ¹³C (CDCl₃*, *75,5 MHz, δ en ppm) : 158,5 (s)* ; *132,0 (s)* ; *130,6 (d) ; 129,1 (s) ; 128,8 (d)* ; *127,1 (d) ; 124,4 (s) ; 123,2 (d) ; 118,8 (d)* ; *117,7 (s)* ; *101,3 (d) ; 55,4 (q) ; 21,9 (t).*

### Stade E : N-[2-(7-méthoxynaphtalén-1-yl)éthyl]acétamide

Dans un réacteur de 8 L sont introduits 136 g de nickel de Raney, 2,06 L d'éthanol et 0,23 L d'eau. Sous agitation à 70 °C et sous 30 bars d'hydrogène, le composé obtenu au Stade D précédent (0,8 kg) en solution dans l'anhydride acétique (2,4 L) est ajouté lentement. A la fin de l'addition, le milieu réactionnel est agité 1 heure sous hydrogène à 30 bars, puis le réacteur est décompressé et les jus sont filtrés. Après concentration du milieu, on cristallise le résidu dans un mélange éthanol / eau 35/65 pour conduire au produit du titre avec un rendement de 89 % et une pureté chimique supérieure à 99 %.
*Point de fusion : 108 °C.*
*Analyse spectroscopique de RMN ¹H (CD₃OD, 300,13 MHz, δ en ppm) : 8,21 (ls, 1H) ; 7,74 (d, J* = *8,9 Hz, 1H) ; 7,65 (d, J = 8,0 Hz, 1H)* ; 7,52 *(d, J* = *2,5 Hz, 1H) ; 7,31-7,2 (m, 2H) ; 7,11 (dd, J = 8,9 et 2,5 Hz, 1H) ; 3,96 (s, 3H) ; 3,52-3,44 (m, 2H) ; 3,23-3,18 (m, 2H) ; 1,94 (s, 3H).*
*Analyse spectroscopique de RMN ¹³C (CD₃OD, 75,5 MHz, δ en ppm) : 173,4 (s) ; 159,4 (s)* ; *135,1 (s) ; 134,6 (s) ; 131,2 (d) ; 130,8 (s)* ; *128,2 (d) ; 127,9 (d) ; 124,2 (d) ; 119,3 (d) ; 103,2 (d) ; 55,9 (q) ; 41,4 (t) ; 34,2 (t) ; 22,6 (q).*

### EXEMPLE 2 : N-[2-(7-méthoxynaphtalén-1-yl)éthyl]acétamide

### Stade A : 4-méthylbenzènesulfonate de 1-[(diméthylamino)méthyl]-7-méthoxy naphtalén-2-yle

A une solution du produit obtenu lors du Stade A de l'Exemple 1 (2,042 g ; 8,84 mmol) dans le diméthylformamide (8 mL), sont ajoutés le *tert*-butylate de potassium (1,091 g; 9,724 mmol) puis, au bout de 5 minutes, le chlorure de tosyle (1,684 g ; 8,84 mmol). Après 6 heures d'agitation, la solution est diluée dans l'acétate d'éthyle, lavée deux fois à l'eau puis à la saumure. La phase organique est séchée sur sulfate de sodium, filtrée et le solvant est évaporé. Le brut obtenu est purifié par chromatographie sur colonne de gel de silice (éluant : acétate d'éthyle/ éther de pétrole, gradient de 30/70 à 70/30) pour donner le produit attendu sous forme d'un solide (1,94 g ; 57 %).
*Point-de fusion : 115-118 °C.*
*Analyse spectroscopique de RMN ¹H (CDCl₃, 300,13 MHz, δ en ppm) : 7,78 (d, J* = *8,1 Hz, 2H) ; 7,7 (d, J* = *9,0 Hz, 1H) ; 7,62 (d, J = 8,8 Hz, 1H) ; 7,53 (d, J* = *2,3 Hz, 1H)* ; *7,34 (d, J = 8,1 Hz, 2H) ; 7,14 (dd, J* = *9,0 et 2,3 Hz, 1H) ; 6,96 (d, J* = *8,8 Hz, 1H) ; 3,93 (s, 3H)* ; *3,67 (s, 2H) ; 2,47 (s, 3H) ; 2,24 (s, 3H).*
*Analyse spectroscopique de RMN ¹³C (CDCl₃, 75,5 MHz, δ en ppm) : 1584 (s) ; 146,9 (s) ; 145,5 (s) ; 135,1 (s) ; 133,2 (s)* ; *130,0 (2 x d) ; 129,9 (d) ; 129,1 (d) ; 128,6 (2 x d) ; 127,8 (s) ; 125,7 (s) ; 118,5 (d) ; 117,9 (d) ; 104,5 (d) ; 55, 3 (q) ; 54,2 (t) ; 45,6 (2 x ; 21,9 (q).*

### Stade B : 4-méthylbenzènesulfonate de 1-(cyanométhyl)-7-méthoxynaphtalén-2-yle

A une solution du produit du Stade A précédent (1,5 g; 9,9 mmol) dans le diméthylformamide (8 mL), est ajouté l'iodométhane (267 µL ; 4,29 mmol). Après 4 heures d'agitation à température ambiante, le cyanure de potassium (304 mg ; 4,68 mmol) est ajouté. La solution est agitée pendant 16 heures supplémentaires puis est diluée dans l'acétate d'éthyle, lavée à l'eau et trois fois à la saumure, séchée sur sulfate de sodium et filtrée. L'évaporation du solvant donne un brut qui est ensuite purifié par chromatographie sur colonne de gel de silice (éluant : acétate d'éthyle / éther de pétrole 40 / 60) pour donner le produit attendu (1,276 g ; 89 %).
*Analyse spectroscopique de RMN ¹H (CDCl₃, 300,13 MHz, δ en ppm) : 7,77 (d, J* = *8,3 Hz, 2H) ; 7,75 (d, J = 8,9 Hz, 1H) ; 7,69 (d, J* = *8,9 Hz, 1H) ; 7,35 (d, J* = *8,3 Hz, 2H) ; 7,22-7,15 (m, 2H) ; 7,01 (d, J = 8,9 Hz, 1H) ; 3,98 (s, 2H) ;* 3,95 *(s, 3H) ; 2,45 (s, 3H).*

### Stade C : 4-méthylbenzènesulfonate de 1-[2-(acétylamino)éthyl]-7-méthoxy naphtalén-2-yle

Dans un flacon scellé, sont introduits le produit du Stade B précédent (54 mg ; 0,147 mmol), le chlorure de nickel hexahydrate (35 mg ; 0,147 mmol), le dichlorométhane (1,5 mL) et le méthanol (1,5 mL). On fait ensuite bullé de l'argon pendant 5 minutes dans la solution, puis le borohydrure de sodium (100 mg ; 2,94 mmol) est ajouté avec précaution par petites portions. Après agitation pendant 30 minutes sous argon et à température ambiante, de l'eau est ajoutée. Au bout de 15 minutes d'agitation, le mélange est filtré sur Célite puis lavé au dichlorométhane. La fraction organique est séchée sur sulfate de sodium puis filtrée. Après évaporation des solvants, le brut obtenu est mis en présence de d'anhydride acétique (1 mL) et d'acétate de sodium (50 mg) sous agitation à température ambiante pendant 30 minutes. Le mélange est versé dans une solution diluée de Na₂CO₃ et le produit est extrait trois fois par de l'acétate d'éthyle. Les fractions organiques sont lavées avec de la saumure, séchées sur sulfate de sodium et filtrées. L'évaporation du solvant donne un brut qui est ensuite purifié par chromatographie sur colonne de gel de silice (éluant : acétate d'éthyle) pour donner le produit attendu sous forme d'un solide (24 mg ; 40 %).
*Analyse spectroscopique de RMN 1H (CDCl₃, 300,13 MHz, δ en ppm) : 7,81 (d, J* = *8,2 Hz, 2H) ; 7,68 (d, J* = *8,9 Hz, 1H) ; 7,62 (d, J* = *2,4 Hz, 1H)* ; *7,55 (d, J* = *8,9 Hz, 1H) ; 7,36 (d, J* = *8,2 Hz, 2H) ; 7,14 (dd, J* = *8,9 et 2,4 Hz, 1H) ; 6,89 (d, J* = *8,9 Hz, 1H) ; 5,97 (m, 1H) ; 4,01 (s, 3H) ; 3,53-3,46 (m, 2H) ; 3,22-3,17 (m, 2H) ; 2,46 (s, 3H) ; 1,95 (s, 3H).*
*Analyse spectroscopique de RMN ¹³C (CDCl₃**, 75,5 MHz, δ* en *ppm) : 170,9 (s) ; 158,9 (s)* ; *146,4 (s)* ; *145,7 (s) ; 134,6 (s) ; 133,3 (s)* ; *130,1 (2 x d) ; 130,0 (d)* ; *128,5 (2 x d) ; 128,2 (d) ; 127,7 (s) ; 126,2 (s) ; 119,2 (d) ; 117,8 (d) ; 103,3 (d) ; 55,8 (q) ; 39,4 (t) ; 26,4 (t) ; 23,4 (q) ; 21,9 (q).*

### Stade D : N-[2-(7-méthoxynaphtalén-1-yl)éthyl]acétamide

Dans un flacon scellé, sont introduits le produit du Stade C précédent (83 mg ; 0,2 mmol), le chlorure de nickel (26 mg ; 0,2 mmol) et le méthanol (4 mL). On fait ensuite bullé de l'argon pendant 5 minutes dans la solution, puis le borohydrure de sodium (136 mg; 4 mmol) est ajouté avec précaution par petites portions. Après agitation pendant 30 minutes sous argon et à température ambiante, le mélange est filtré sur Célite puis lavé à l'acétate d'éthyle et les solvants sont évaporés. Le brut obtenu est purifié par chromatographie sur colonne de gel de silice (éluant : acétate d'éthyle) pour donner le produit attendu (39 mg ; 80 %).
*Analyse spectroscopique de RMN ¹H (CD₃OD, 300,13 MHz, δ en ppm)* : *8,21 (ls, 1H) ; 7,74 (d, J* = *8,9 Hz, 1H) ; 7,65 (d, J* = *8,0 Hz, 1H) ; 7,52 (d, J* = *2,5 Hz, 1H) ; 7,31-7,2 (m, 2H)* ; *7,11 (dd, J* = *8,9 et 2,5 Hz, 1H) ; 3,96 (s, 3H) ; 3,52-3,44 (m, 2H) ; 3,23-3,18 (m, 2H)* ; *1,94 (s, 3H).*
*Analyse spectroscopique de RMN ¹³C (CD₃OD, 75,5 MHz, δ en ppm) : 173,4 (s) ; 159,4 (s) ; 135,1 (s)* ; *134,6 (s) ;* 131,2 *(d) ; 130,8 (s) ; 128,2 (d) ; 127,9 (d) ; 124,2 (d)* ; *119,3 (d)* ; *103,2 (d)* ; *55,9 (q) ; 41,4 (t) ; 34,2 (t) ; 22,6 (q).*

### EXEMPLE 3 : N-[2-(7-méthoxynaphtalén-1-yl)éthyl]acétamide

### Stade A : 8-méthoxynaphto[2,1-b]furan-2(1H)-one

Une solution aqueuse à 85 % d'hydroxyde de potassium (2,76 g ; 40 mmol) et de 7-méthoxy-naphtalén-2-ol (6,96 g ; 40 mmol) dans l'eau (80 mL) est ajoutée goutte à goutte à température ambiante à une solution de glyoxal (40 % dans l'eau ; 28 mL ; 240 mmol). Après 3 heures d'agitation, le précipité blanc est collecté par filtration et lavé à l'eau. Le solide (le 8-méthoxy-1,2-dihydronaphto[2,1-*b*]furane-1,2-diol) obtenu est mis en solution dans du 1,2-dichloroéthane (160 mL) et une solution aqueuse de HCl 3 M (300 mL) est ensuite ajoutée. Le mélange hétérogène est chauffé à 50 °C sous agitation vigoureuse. Au bout de 1,5 heures, tout le solide est dissous et les deux phases sont séparées. La phase organique est collectée et les solvants sont évaporés. Le brut est séché par azéotropie avec du toluène pour donner le produit du titre (8,69 g) qui sera utilisé directement pour l'étape suivante sans plus de purification.
*Analyse spectroscopique de RMN ¹H (DMSO-d₆, 300,13 MHz, δ en ppm) : 7,88 (d, J* = *8,8 Hz, 1H) ; 7,85 (d, J* = *8,6 Hz, 1H) ; 7,28 (d, J* = *8,8 Hz, 1H) ; 7,12-7,06 (m, 2H)* ; *4,15 (s, 2H) ; 3,89 (s, 3H).*

### Stade B : N, N-dibenzyl-2-(2-hydroxy-7-méthoxynaphtalén-1-yl)acétamide

Dans un ballon, sont introduits le produit du Stade A précédent (1,976 g ; 9,23 mmol) et la dibenzylamine (4 mL ; 20,3 mmol) pour être ensuite chauffés à 120 °C pendant 2 heures. Après refroidissement, le résidu est dilué avec de l'acétate d'éthyle (200 mL). L'addition d'une solution aqueuse de HCl 2 M précipite le sel de chlorhydrate de dibenzylamine qui est ensuite filtré sur Célite. La phase organique est lavée avec une solution aqueuse de HCl 2 M, puis lavée avec de la saumure, séchée sur sulfate de sodium et filtrée. Après évaporation des solvants, le brut obtenu est purifié par chromatographie sur colonne de gel de silice (éluant : acétate d'éthyle / éther de pétrole 30 / 70) pour donner le produit attendu sous forme d'un solide (2,88 g ; 76 %)
*Point de fusion : 155-157 °C.*
*Analyse spectroscopique de RMN ¹H (CDCl₃, 300,13 MHz, δ en ppm) : 9,85 (ls, 1H)* ; *7,66 (d, J* = *8,7 Hz, 1H); 7,62 (d, J* = *8,7 Hz, 1H); 7,4-7,24 (m, 6H) ; 7,18-7,14 (m, 4H)* ; *7,08 (d, J* = *8,7 Hz, 1H)* ; *6,95-6,91 (m, 2H) ; 4, 72 (s, 2H) ; 4,64 (s, 2H)* ; *4,21 (s, 2H)* ; *3,55 (s, 3H).*
*Analyse sprectroscopique de RMN ¹³C (CDCl₃, 75,5 MHz, δ en ppm) : 174,6 (s); 158,6 (s) ; 155,8 (s)* ; *136,4 (s) ; 135,5 (s) ; 134,1 (s) ; 130,6 (d) ; 129,3 (d) ; 129,2 (2 x d)* ; *128,9 (2 x d) ; 128,4 (2 x d) ; 128,1 (d)* ; *127,8 (d) ; 126,4 (2 x d) ; 124,6 (s) ; 117,5 (d)* ; *114,9 (d) ; 111,5 (s) ; 101,2 (d) ; 55,9 (q)* ; *50,8 (t) ; 49,0 (t) ; 31,5 (t).*

### Stade C : 1-[2-(dibenzylamino)éthyl]-7-méthoxynaphtalén-2-ol

A une solution du produit du Stade B précédent (230 mg ; 0,56 mmol) dans le tétrahydrofurane (10 mL), est ajoutée une solution 1 M de complexe borane-tétrahydrofurane dans le tétrahydrofurane (1,7 mL ; 1,7 mmol). Le mélange est chauffé au reflux pendant 2 heures puis est ajoutée une solution aqueuse de HCl 2 M (10 mL). Après agitation toute la nuit, une solution saturée de NaHCO₃ est additionnée jusqu'à atteindre un pH neutre et le produit est extrait trois fois avec de l'acétate d'éthyle. Les fractions organiques sont séchées sur sulfate de sodium, filtrées et le solvant est évaporé. Le brut obtenu est purifié par chromatographie sur colonne de gel de silice (éluant : acétate d'éthyle / éther de pétrole 30 / 70) pour donner le produit attendu (150 mg ; 72 %).
*Analyse spectroscopique de RMN ¹H (CDCl₃, 300,13 MHz, δ en ppm) : 12,09 (ls, 1H) ; 7,69 (d, J* = *8,9 Hz, 1H) ; 7,61 (d, J* = *8,8 Hz, 1H) ; 7,44-7,26 (m, 10H)* ; *7,14 (d, J* = *8,8 Hz, 1H) ; 7,12 (d, J* = *2,4 Hz, 1H)* ; *7,0 (dd, J = 8,8 et 2,4 Hz, 1H) ; 3,93 (s, 3H) ; 3, 79 (s, 4H) ; 3,21-3,18 (m, 2H) ; 3,01-2,98 (m, 2H).*

### Stade D : trifluorométhanesulfonate de 1-[2-(dibenzylamino)éthyl]-7-méthoxy naphtalén-2-yle

A une solution du produit du Stade C précédent (303 mg ; 0,763 mmol) dans le dichlorométhane (10 mL), est ajouté à 0 °C l'anhydride triflique (135 µL ; 0,801 mmol). Au bout de 2 heures d'agitation à cette température, le solvant est évaporé. Le résidu est repris dans un mélange diéthyléther / solution aqueuse semi-saturée de NaHCO₃. Après séparation, la phase organique est séchée sur sulfate de sodium et filtrée. Après évaporation des solvants, le brut obtenu est purifié par chromatographie sur colonne de gel de silice (éluant : acétate d'éthyle / éther de pétrole 10 / 90) pour donner le produit attendu (306 mg ; 76 %).
*Analyse spectroscopique de RMN ¹H (CDCl₃, 300,13 MHz, δ en ppm)* : *7,73 (d, J* = *8,9 Hz, 1H) ; 7,69 (d, J* = *8,9 Hz, 1H) ; 7,44-7,4 (m, 4H) ; 7,35-7,22 (m, 7H) ; 7,15 (dd, J* = *8,9 et 2,3 Hz, 1H) ; 6,99 (d, J* = *2,3 Hz, 1H) ; 3,78 (s, 4H) ; 3,6 (s, 3H) ; 3,43-3,37 (m, 2H) ; 2,91-2,86 (m, 2H).*
*Analyse spectroscopique de RMN ¹³C (CDCl₃, 75,5 MHz, δ en ppm) : 158,9 (s)* ; *146,1 (s) ; 139,8 (2 x s) ; 134,3 (s)* ; *130,3 (d)* ; *128,7 (4 x d)* ; *128,6 (d) ; 128,3 (4 x d) ; 128,1 (s) ; 127,4 (s) ; 127,0 (2 x d) ; 119,5 (d)* ; *118,7 (s, J_{C-F}* = *318 Hz) ; 116,8 (d) ; 102,8 (d) ; 58,3 (2 x t) ; 55,3 (q) ; 52,5 (t) ; 24,6 (t).*

### Stade E: N,N-dibenzyl-2-(7-méthoxynaphtalén-1-yl)éthanamine

Dans un flacon, sont introduits le produit du Stade D précédent (130 mg ; 0,25 mmol), l'acétate de palladium (5,6 mg ; 0,025 mmol), la triphénylphosphine (20 mg ; 0,075 mmol), le formiate d'ammonium (142 mg ; 2,25 mmol) et le diméthylformamide (1 mL). Après 16 heures d'agitation à 60 °C, la solution est diluée dans l'acétate d'éthyle, lavée à l'eau, lavée deux fois à la saumure, séchée sur sulfate de sodium et filtrée. Après évaporation des solvants, le brut obtenu est purifié par chromatographie sur colonne de gel de silice (éluant : acétate d'éthyle / éther de pétrole 10/90) pour donner le produit du titre sous forme d'un solide blanc (93 mg ; 98 %).
*Point de fusion : 92-93 °C.*
*Analyse spectroscopique de RMN ¹H (CHCl₃*, *300, 13 MHz, δ en ppm)*: *7,74 (d, J* = *8,9 Hz, 1H) ; 7,65 (m, 1H); 7,43-7,39 (m, 4H) ; 7,35-7,22 (m, 8H) ; 7,11 (dd, J* = *8,9 et 2,4 Hz, 1H) ; 7,05 (d, J* = *2,4 Hz, 1H) ; 3,76 (s, 4H) ; 3,67 (s, 3H) ; 3,29-3,23 (m, 2H) ; 2,92-2,86 (m, 2H).*
*Analyse spectroscopique de RMN ¹³C (CDCl₃, 75,5 MHz, δ en ppm) : 157,6 (s) ; 139,9 (2 x s) ; 135,3 (s) ; 133,2 (s)* ; *130,3 (d)* ; *129,3 (s)* ; *128,7 (4 x d) ; 128,3 (4 x d) ; 127,3 (d)* ; *127,0 (2 x d) ; 126,5 (d)* ; *123,3(d)* ; *118,2 (d)* ; *102,2 (d) ; 58,6 (2 t) ; 55,2 (q) ; 54,2 (t)* ; *31,5 (t).*

### Stade F : N-[2-(7-méthoxynaphtalén-1-yl)éthyl]acétamide

Dans un flacon placé dans un autoclave, sont introduits le produit du Stade E précédent (66 mg ; 0,173 mmol), l'hydroxyde de palladium sur charbon (20 % Pd, 60 % d'humidité ; 10 mg), l'éthanol (2 mL) et l'acétate d'éthyle (2 mL). L'autoclave est rempli d'hydrogène sous pression (5 bars) et le mélange est agité pendant 30 heures. La solution est ensuite filtrée sur Célite, lavée à l'éthanol et les solvants sont évaporés. Sur le brut réactionnel, sont ajoutés l'anhydride acétique (500 µL) et l'acétate de sodium (100 mg). Au bout de 4 heures d'agitation, le mélange est dilué dans l'acétate d'éthyle. La phase organique est lavée deux fois avec une solution aqueuse de soude 2 M, de la saumure, séchée sur sulfate de sodium et filtrée. Après évaporation des solvants, le produit attendu est obtenu pur (41 mg ; 98 %).
*Analyse spectroscopique de RMN ¹H (CD₃OD, 300,13 MHz, δ en ppm) : 8,21 (ls, 1H)* ; *7,74 (d, J* = *8,9 Hz, 1H) ; 7,65 (d, J* = *8,0 Hz, 1H) ; 7,52 (d, J* = *2,5 Hz, 1H) ; 7,31-7,2 (m, 2H) ; 7,11 (dd, J* = *8,9 et 2,5 Hz, 1H) ; 3,96 (s, 3H) ; 3,52-3,44 (m, 2H) ; 3,23-3,18 (m, 2H) ; 1,94 (s, 3H).*
*Analyse spectroscopique de RMN ¹³C (CD₃OD, 75,5 MHz, δ en ppm) : 173,4 (s) ; 159,4 (s)* ; *135,1 (s)* ; *134,6 (s) ; 131,2 (d) ; 130,8 (s)* ; *128,2 (d) ; 127,9 (d)* ; *124,2 (d)* ; *119,3 (d)* ; *103,2 (d)* ; *55,9 (g) ; 41,4 (t) ; 34,2 (t) ; 22,6 (q).*

### EXEMPLE 4 : N-[2-(7-méthoxynaphtalén-1-yl)éthyl]acétamide

### Stade A : 1-(2-hydroxyéthyl)-7-méthoxynaphtalén-2-ol

Le produit obtenu lors du Stade A de l'Exemple 3 (8,96 g ; 40 mmol) est dissous dans le tétrahydrofurane (160 mL) puis l'hydrure de lithium et d'aluminium (1,52 ; 40 mmol) est ajouté par portions à 0 °C sous flux d'azote. Le mélange est agité pendant 16 heures à température ambiante puis la réaction est stoppée à 0 °C avec l'ajout d'acétate d'éthyle puis d'eau. Une solution aqueuse d'acide sulfurique 1 M (80 mL) est additionnée. Au bout d'une heure d'agitation, le mélange hétérogène est filtré sur Célite et lavé à l'acétate d'éthyle. Après décantation et séparation, la phase organique est lavée à l'eau puis avec de la saumure. La solution est à nouveau filtrée à travers une fine couche de silice et les solvants sont évaporés pour conduire au produit du titre sans plus de purification (8,21 g ; 94 % à partir du 7-méthoxy-naphtalén-2-ol).
*Analyse spectroscopique de RMN ¹H (DMSO-d₆, 300,13 MHz, δ en ppm) : 7,6 (d, J* = *8,8 Hz, 1H) ; 7,49 (d, J* = *8,8 Hz, 1H) ; 7,25 (d, J* = *2,4 Hz, 1H) ; 6,94 (d, J* = *8,8 Hz, 1H)* ; *6,9 (dd, J* = *8,8 et 2,4 Hz, 1H) ; 3,9 (s, 3H) ; 3,79-3,73 (m, 2H) ; 3,31-3,25 (m, 2H).*

### Stade B : 4-méthylbenzènesulfonate de 7-méthoxy-1-(2-{[(4-méthylphényl)sulfonyl] oxy}éthyl)naphtalén-2-yle

Dans un ballon, sont introduits le produit du Stade A précédent (436 mg ; 2 mmol), le dichlorométhane (10 mL), la triéthylamine (670 µL ; 4,8 mmol) et la 4-diméthylaminopyridine (12 mg ; 0,1 mmol). Après avoir refroidi à 0 °C, le chlorure de tosyle (800 mg ; 4 mmol) est ajouté en une fois et la solution est agitée pendant 2 heures à 0 °C puis 14 heures à température ambiante. Après évaporation du solvant, le résidu est repris dans de l'acétate d'éthyle et l'eau. La fraction organique est lavée à l'eau et avec de la saumure, séchée sur sulfate de sodium et filtrée, Une fois le solvant évaporé, le brut obtenu est purifié par chromatographie sur colonne de gel de silice (éluant : acétate d'éthyle / éther de pétrole, gradient de 20/80 à 30/70) pour donner le produit du titre (728 mg ; 69 %).
*Analyse spectroscopique de RMN ¹H (CDCl₃, 300,13 MHz, δ en ppm) : 7,78 (d, J* = *8,3 Hz, 2H) ; 7,69 (d, J* = *9,1 Hz, 1H)* ; *7,66 (d, J* = *8,3 Hz, 2H) ; 7,59 (d, J* = *8,9 Hz, 1H) ; 7,35 (d, J* = *8,3 Hz, 2H) ; 7,26 (d, J* = *2,4 Hz, 1H) ; 7,23 (d, J* = *8,3 Hz, 2H) ; 7,15 (dd, J* = *8,9 et 2,4 Hz, 1H) ; 7,03 (d, J* = *9,1 Hz, 1H); 4,2 (t, J* = *7,7 Hz, 2H); 3,94 (s, 3H); 3,34 (d, J* = *7,7 Hz, 2H) ; 2,47 (s, 3H); 2,39 (s, 3H).*

### Stade C : 4-méthylbenzènesulfonate de 1-[2-(acétylamino)éthyl]-7-méthoxy naphtalén-2-yle

Dans un flacon scellé, sont introduits le produit du Stade B précédent (124 mg ; 0,236 mmol), l'acétonitrile (1 mL) et une solution aqueuse d'ammoniaque à 35 % (1 Le flacon est placé dans un bain chauffé à 110°C et la solution est agitée pendant 3,5 heures. Après refroidissement, la solution est diluée dans l'acétate d'éthyle, lavée avec une solution diluée de NaHCO₃, lavée avec de la saumure, séchée sur sulfate de sodium et filtrée. Une fois les solvants évaporés, le brut est dissous dans l'anhydride acétique (1 mL) et l'acétate de sodium (300 mg) est ensuite ajouté. Après 14 heures d'agitation, le mélangé est versé dans une solution diluée de NaHCO₃. Au bout de 30 minutes d'agitation, le produit est extrait trois fois à l'acétate d'éthyle. La phase organique est lavée à l'eau et avec de la saumure, séchée sur sulfate de sodium et filtrée. Après évaporation des solvants, le brut est purifié par chromatographie sur colonne de gel de silice (éluant : acétate d'éthyle) pour donner le produit du titre (84 mg ; 86 %).
*Analyse spectroscopique de RMN ¹H (CDCl₃, 300,13 MHz, δ en ppm) : 7,81 (d, J* = *8,2 Hz, 2H) ; 7,68 (d, J* = *8,9 Hz, 1H) ; 7,62 (d, J* = *2,4 Hz, 1H); 7,55 (d, J* = *8,9 Hz, 1H) ; 7,36 (d, J* = *8,2 Hz, 2H) ; 7,14 (dd, J* = *8,9 et 2,4 Hz, 1H) ; 6,89 (d, 8,9 Hz, 1H) ; 5,97 (m, 1H)* ; *4,01 (s, 3H) ; 3,53-3,46 (m, 2H) ; 3,22-3,17 (m, 2H) ; 2,46 (s, 3H) ; 1,95 (s, 3H).*
*Analyse spectroscopique de RMN ¹³C (CDCl₃, 75,5 MHz, δ en ppm) : 170,9 (s) ; 158,9(s)* ; *146,4 (s) ; 145, 7 (s) ; 134,6 (s) ; 133,3 (s) ; 130,1 (2 x d) ; 130,0 (d)* ; *128,5 (2 x d) ; 128,2 (d) ; 127,7 (s) ; 126,2 (s) ; 119,2 (d) ; 117,8 (d) ; 103,3 (d) ; 55,8 (q) ; 39,4 (t) ; 26,4 (t)* ; *23,4 (q) ; 21,9 (q).*

### Stade D : N-[2-(7-méthoxynaphtalén-1-yl)éthyl]acétamide

Le produit du titre est obtenu selon le procédé décrit dans le Stade D de l'Exemple 2 en utilisant le produit du Stade C précédent comme réactif de départ.
*Analyse spectroscopique de RMN ¹H (CD₃OD, 300,13 MHz, δ en ppm) : 8,21 (ls, 1H)* ; *7,74 (d, J* = *8,9 Hz, 1H) ; 7,65 (d, J* = *8,0 Hz, 1H) ; 7,52 (d, J* = *2,5 Hz, 1H) ; 7,31-7,20 (m, 2H) ; 7,11 (dd, J* = *8,9 et 2,5 Hz, 1H) ; 3,96 (s, 3H) ; 3,52-3,44 (m, 2H) ; 3,23-3,18 (m, 2H) ; 1,94 (s, 3H).*
*Analyse spectroscopique de RMN ¹³C (CD₃OD, 75,5 MHz, δ en ppm) : 173,4 (s) ; 159,4 (s)* ; *135,1 (s) ; 134,6 (s) ; 131,2 (d)* ; *130,8 (s) ; 128,2 (d)* ; *127,9 (d)* ; *124,2 (d) ; 119,3 (d)* ; *103,2 (d) ; 55,9 (q) ; 41,4 (t) ; 34,2 (t) ; 22,6 (q).*

### EXEMPLE 5 : N-[2-(7-méthoxynaphtalén-1-yl)éthyl]acétamide

### Stade A : propane-1-sulfonate de 7-méthoxy-1-{2-[(propylsulfonyl)oxy]éthyl} naphtalén-2-yle

Le produit obtenu lors du Stade A de l'Exemple 4 (2,45 g ; 11,238 mmol) est mis en solution dans le dichlorométhane (60 mL) et la triéthylamine (3,7 mL ; 26,4 mmol) est ensuite additionnée. Après refroidissement à 0 °C, le chlorure de *n*-propylsulfonyle (2,8 mL ; 24,6 mmol) est ajouté goutte à goutte. Au bout de 2 heures d'agitation à température ambiante, le solvant est évaporé. Le résidu obtenu est repris dans du diéthyléther et de l'eau. Après séparation, la phase organique est lavée avec une solution aqueuse diluée de HCl, avec de l'eau et avec de la saumure, séchée sur sulfate de sodium et filtrée. Une fois le solvant évaporé, le brut obtenu est purifié par chromatographie sur colonne de gel de silice (éluant : acétate d'éthyle / éther de pétrole 20 / 80) pour donner le produit du titre sous forme d'un solide (3,335 g ; 66 % sur 3 étapes à partir du 7-méthoxy-naphtalén-2-ol).
*Analyse spectroscopique de RMN ¹H (CDCl₃, 300,13 MHz, δ en ppm) : 7,72 (m, 2H) ; 7,37 (d, J* = *2,4 Hz, 1H) ; 7,31 (d, J* = *9,0 Hz, 1H) ; 7,17 (dd, J* = *9,0 et 2,4 Hz, 1H) ; 4,48 (t, J* = *8,0 Hz, 2H) ; 3,96 (s, 3H) ; 3,6 (t, J* = *8,0 Hz, 2H) ; 3,45-3,4 (m, 2H) ; 3,04-2,98 (m, 2H) ; 2,1 (m, 2H) ; 1,8 (m, 2H) ; 1,17 (t, J* - *7,3 Hz, 3H) ; 0,99 (t, J* = *7,3 Hz, 3H).*

### Stade B : propane-1-sulfonate de 1-[2-(acétylamino)éthyl]-7-méthoxynaphtalén-2-yle

Dans un flacon scellé, sont introduits le produit du Stade A précédent (532 mg ; 1,237 mmol), l'acétonitrile (18 mL) et une solution aqueuse d'ammoniaque à 35 % (18 mL). Le mélange est ensuite placé dans un bain chauffé à 110 °C pendant 3 heures. Les solvants sont ensuite évaporés sous pression réduite en réalisant un azéotrope avec l'éthanol. Le produit brut est solubilisé dans l'anhydride acétique (5 mL) et, dans un deuxième temps, l'acétate de sodium (500 mg) est ajouté. Au bout d'une heure d'agitation, la solution est diluée avec de l'acétate d'éthyle et est ensuite versée avec précaution dans une solution aqueuse saturée de NaHCO₃. Après 15 minutes d'agitation, les deux phases sont séparées et la phase aqueuse est extraite deux fois avec de l'acétate d'éthyle. Les fractions organiques sont rassemblées, lavées avec de l'eau puis avec de la saumure, séchées sur sulfate de sodium et filtrées. Une fois le solvant évaporé, le brut obtenu est purifié par chromatographie sur colonne de gel de silice (éluant : acétate d'éthyle) pour donner le produit du titre (289 mg ; 64 %).
*Analyse spectroscopique de RMN ¹H (CDCl₃, 300,13 MHz, δ en ppm) : 7,73 (d, J* = *8,9 Hz, 1H); 7,68 (d, J* = *8,9 Hz, 1H) ; 7,62 (d, J* = *2,5 Hz, 1H) ; 7,27 (d, J* = *8,9 Hz, 1H) ; 7,16 (dd, J* = *8,9 et 2,5 Hz, 1H) ; 5,95 (m, 1H) ; 4,02 (s, 3H) ; 3,62-3,54 (m, 2H) ; 3,43-3,32 (m, 2H) ; 2,09 (m, 2H) ; 1,17 (t, J* = *7,4 Hz, 3H).*
*Analyse spectroscopique de RMN ¹³C (CDCl₃, 75,5 MHz, δ en ppm) : 170,9 (s)* ; *159,0 (s)* ; *145,6 (s) ; 134,7 (s) ; 130,2 (d) ; 128,6 (d) ; 127,7 (s) ; 126,0 (s) ; 119,2 (d)* ; *118,1 (d) ; 103,3 (d)* ; *55,9 (g) ; 53,6 (t) ; 39,4 (t) ; 26,5 (t) ; 23,4 (q) ; 17,6 (t)* ; *13,1 (q).*

### Stade C : N-[2-(7-méthoxynaphtalén-1-yl)éthyl]acétamide

Dans un ballon, sont introduits le produit du Stade B précédent (327 mg ; 0,896 mmol), l'acétate d'ammonium (1,19 g ; 15,54 mmol), le palladium à 10 % sur charbon (95 mg ; 0,09 mmol), l'éthanol (3 mL) et le magnésium en poudre (83 mg ; 3,584 mmol). Au bout de 16 heures d'agitation, le mélange hétérogène est filtré sur Célite et lavé avec de l'acétate d'éthyle. Après évaporation des solvants, le brut obtenu est purifié par chromatographie sur colonne de gel de silice (éluant : acétate d'éthyle) pour donner le produit du titre (190 mg ; 87%).
*Analyse spectroscopique de RMN ¹H (CD₃OD, 300,13 MHz, δ en ppm) : 8,21 (ls, 1H) ; 7,74 (d, J* = *8,9 Hz, 1H) ; 7,65 (d, J* = *8,0 Hz, 1H) ; 7,52 (d, J* = *2,5 Hz, 1H) ; 7,31-7,2 (m, 2H) ; 7,11 (dd, J* = *8,9 et 2,5 Hz, 1H) ; 3,96 (s, 3H) ; 3,52-3,44 (m, 2H) ; 3,23-3,18 (m, 2H) ; 1,94 (s, 3H).*
*Analyse spectroscopique de RMN ¹³C (CD₃OD, 75,5 MHz, δ en ppm) : 173,4 (s) ; 159,4 (s)* ; *135,1 (s)* ; *134,6 (s)* ; *131,2 (d) ; 130,8 (s) ; 128,2 (d) ; 127,9 (d) ; 124,2 (d)* ; *119,3 (d)* ; *103,2 (d) ; 55,9 (q) ; 41,4 (t) ; 34,2 (t) ; 22,6 (q).*

### EXEMPLE 6 : N-[2-(7-méthoxynaphtalén-1-yl)éthyl]acétamide

### Stade A : propane-1-sulfonate de 1-[2-(2,5-dioxopyrrolidin-1-yl)éthyl]-7-méthoxy naphtalén-2-yle

Dans un ballon, sont introduits le produit obtenu lors du Stade A de l'Exemple 5 (240 mg ; 0,558 mmol), le carbonate de potassium (231 mg ; 1,674 mmol), le succinimide (66 mg ; 0,67 mmol) et le diméthylformamide (2 Après agitation pendant 16 heures à 100 °C, la solution est diluée dans l'acétate d'éthyle, lavée avec une solution aqueuse diluée de HCl, avec de l'eau et avec de la saumure, puis séchée sur sulfate de sodium et filtrée. Une fois le solvant évaporé, le brut obtenu est purifié par chromatographie sur colonne de gel de silice (éluant : acétate d'éthyle / éther de pétrole 70 / 30) pour conduire au produit du titre (123 mg ; 57 %).
*Analyse spectroscopique de RMN ¹H (CDCl₃, 300,13 MHz, δ en ppm) : 7,74 (d, J* = *9,0 Hz, 1H) ; 7,7 (d, J* = *8,9 Hz, 1H) ; 7,6 (d, J* = *2,4 Hz, 1H) ; 7,38 (d, J* = *9,0 Hz, 1H) ; 7,16 (dd, J* = *8,9 et 2,4 Hz, 1H) ; 4,05 (s, 3H) ; 3,86-3,8 (m, 2H) ; 3,58-3,52 (m, 2H) ; 3,33-3,27 (m, 2H)* ; *2,72 (s, 4H) ; 2,14 (m, 2H) ; 1,2 (t, J* = *7,5 Hz, 3H).*
*Analyse spectroscopique de RMN ¹³C (CDCl₃, 75,5 MHz, δ en ppm) : 177,3 (2 x s) ; 159,1 (s) ; 145,3 (s) ; 134,4 (s) ; 130,4 (d) ; 128,8 (d) ; 127,7 (s) ; 124,2 (s) ; 119,2 (d) ; 118,4 (d) ; 102,4 (d) ; 55,8 (q)* ; *53,5 (t) ; 37,7 (t) ; 28,4 (2 x t) ; 24,7 (t) ; 17,5 (t) ; 13,1 (q).*

### Stade B : N-[2-(7-méthoxynaphtalén-1-yl)éthyl]acétamide

Dans un ballon, sont introduits le produit du Stade A précédent (65 mg ; 0,16 mmol), l'acétate d'ammonium (240 mg ; 3,12 mmol), le palladium à 10 % sur charbon (16,5 mg ; 0,016 mmol), le méthanol (0,7 mL) et du magnésium en poudre (8 mg ; 0,36 mmol) en une seule portion. Après agitation pendant 12 heures à 30 °C, le mélange hétérogène est filtré sur Célite puis lavé à l'acétate d'éthyle. Après évaporation des solvants, le brut est dissous dans l'éthanol (2 mL) dans un tube scellé puis une solution aqueuse de soude (180 mg ; 4,5 mmol dans 1 mL d'eau) est ajoutée. Le mélange est ensuite dilué dans l'anhydride acétique (5 mL) et l'acétate de sodium (500 mg) est introduit. Au bout d'une heure d'agitation, la solution est diluée dans l'acétate d'éthyle pour être versée avec précaution dans une solution aqueuse saturée de NaHCO₃. Après 15 minutes d'agitation, les deux phases sont séparées et la phase aqueuse est extraite deux fois par de l'acétate d'éthyle. Les fractions organiques sont rassemblées, lavées à l'eau et à la saumure, séchées sur sulfate de sodium et filtrées. Une fois le solvant évaporé, le brut obtenu est purifié par chromatographie sur colonne de gel de silice (éluant : acétate d'éthyle) pour donner le produit du titre (14,4 mg ; 37 %).
*Analyse spectroscopique de RMN ¹H (CD₃OD, 300,13 MHz, δ en ppm) : 8,21 (ls, 1H)* ; *7,74 (d, J* = *8,9 Hz, 1H) ; 7,65 (d, J* = *8,0 Hz, 1H) ; 7,52 (d, J* = *2,5 Hz, 1H) ; 7,31-7,2 (m, 2H) ; 7,11 (dd, J* = *8,9 et 2,5 Hz, 1H) ; 3,96 (s, 3H) ; 3,52-3,44 (m, 2H) ; 3,23-3,18 (m, 2H)* ; *1,94 (s, 3H).*
*Analyse spectroscopique de RMN ¹³C (CD₃OD, 75,5 MHz, δ en ppm) : 173,4 (s) ; 159,4 (s) ; 135,1 (s)* ; *134,6 (s) ; 131,2 (d) ; 130,8 (s) ; 128,2 (d) ; 127,9 (d)* ; *124,2 (d) ; 119,3 (d) ; 103,2 (d)* ; *55,9 (q) ; 41,4 (t) ; 34,2 (t) ; 22,6 (q).*

### EXEMPLE 7: N-[2-(7-méthoxynaphtalén-1-yl)éthyl]acétamide

### Stade A : 4-méthylbenzènesulfonate de 1-(2-hydroxyéthyl)-7-méthoxynaphtalén-2-yle

A une solution du produit obtenu lors du Stade A de l'Exemple 4 (109 mg ; 0,5 mmol) dans le tétrahydrofurane (2,5 mL), sont ajoutés à 0 °C le *tert*-butylate de potassium (56,4 mg ; 0,5 mmol) puis, au bout de 5 minutes, le chlorure de tosyle (95 mg ; 0,5 mmol). Au bout de 3 heures d'agitation, la solution est laissée revenir à température ambiante et est agitée pendant 15 heures supplémentaires. Le solvant est évaporé et le brut obtenu est purifié par chromatographie sur colonne de gel de silice (éluant : acétate d'éthyle / éther de pétrole 50 / 50) pour donner un mélange de deux diastéréoisomères (116 mg ; 62 %) dans un ratio 88:12. Le mélange est utilisé tel quel sans autre purification.
*Analyse spectroscopique de RMN ¹H (CDCl₃, 300,13 MHz, δ en ppm) : 7,83 (d, J* = *8,2 Hz, 2H) ; 7,71 (d, J* = *9,1 Hz, 1H) ; 7,59 (d, J* = *8,9 Hz, 1H) ; 7,35 (d, J* = *8,2 Hz, 2H) ; 7,29 (d, J* = *2,4 Hz, 1H) ; 7,15 (dd, J* = *8,9 et 2,4 Hz, 1H)* ; *7,04 (d, J* = *9,1 Hz, 1H) ; 3,91 (s, 3H) ; 3,87 (t, J* = 7,1 *Hz, 2H) ; 3,23 (t, J* = *7,1 Hz, 2H)* ; *2,46 (s, 3H).*
*Analyse spectroscopique de RMN ¹³C (CDCl₃, 75,5 MHz, δ en ppm) : 158,6 (s) ; 146,7 (s) ; 145,7 (s) ; 134,5 (s) ; 133,3 (s) ; 130,3 (d) ; 130,1 (2 x d) ; 128,5 (2 x d) ; 128,2 (d) ; 127,8 (s) ; 125,7 (s)* ; *118,5 (d) ; 118,3 (d) ; 103,4 (d) ; 62,1 (t) ; 55,5 (q) ; 29,7 (t) ; 21,9 (q).*

### Stade B : 2-(7-méthoxynaphtalén-1-yl)éthanol

Dans un flacon scellé, sont introduits le mélange de diastéréoisomères obtenu dans le Stade A précédent (80 mg ; 0,176 mmol du bon isomère), le chlorure de nickel hexahydrate (51 mg ; 0,215 mmol), le dichlorométhane (2 mL) et le méthanol (2 On fait ensuite bullé de l'argon pendant 5 minutes dans la solution, puis le borohydrure de sodium (146 mg ; 4,3 mmol) est ajouté avec précaution par petites portions. Au bout d'une heure d'agitation à température ambiante sous argon, une solution aqueuse diluée de HCl est additionnée. Après 4 heures d'agitation, le mélange est filtré sur Célite et lavé à l'éthanol. Le solvant est évaporé et le brut obtenu est purifié par chromatographie sur colonne de gel de silice (éluant : acétate d'éthyle / éther de pétrole 40 / 60) pour donner le produit du titre (22 mg ; 62 %).
*Analyse spectroscopique de RMN ¹H (CDCl₃, 300,13 MHz, δ en ppm) : 7,72 (d J* = *9,1 Hz, 1H) ; 7,63 (d, J* = *8,1 Hz, 1H) ; 7,3-7,21 (m, 3H) ; 7,13 (dd, J* = *9,1 et 2,6 Hz, 1H) ; 3,93 (t, J* = *6, 7 Hz, 2H) ; 3,88 (s, 3H) ; 3,24 (d, J* = *6,7 Hz, 2H) ; 1,99 (ls, 1H).*
*Analyse spectroscopique de RMN ¹³C (CDCl₃, 75,5 MHz, δ en ppm) : 157,8 (s)* ; *133,2 (s)* ; *133,0 (s) ; 130,4 (d) ; 129,4 (s) ; 127,7 (d) ; 127,1 (d) ; 123,3 (d) ; 118,1 (d) ; 102,5 (d) ; 62,7 (t) ; 55,4 (q)* ; *36,4 (t).*
*Spestrométrie de masse (ESI; m*/*z(*%*)) : 202(29) [M]*⁺*^{•}; 171(100).*

### Stade C : méthanesulfonate de 2-(7-méthoxynaphtalén-1-yl)éthyle

A une solution du produit du Stade B précédent (275 mg ; 1,361 mmol) dans le dichlorométhane (7 mL), sont ajoutés à 0 °C la triéthylamine (227 µL ; 1,633 mmol) et le chlorure de méthanesulfonyle (116 µL ; 1,498 mmol). Au bout d'une heure d'agitation, les solvants sont évaporés et le résidu est repris dans du diéthyléther et de l'eau. Après séparation, la phase organique est lavée trois fois à l'eau et avec de la saumure, séchée sur sulfate de sodium et filtrée. L'évaporation du solvant fournit le produit attendu propre sans autre purification supplémentaire (356 mg ; 93 %).
*Analyse spectroscopique de RMN ¹H (CDCl₃, 300,13 MHz, δ en ppm) : 7,72 (d, J* = *9,1 Hz, 1H) ; 7,66 (d, J* = *8,9 Hz, 1H) ; 7,31-7,21 (m, 3H) ; 7,13 (dd, J* = *9,1 et 2,5 Hz, 1H) ; 4,47 (t, J* = *7,4 Hz, 2H) ; 3,92 (s, 3H)* ; *3,45 (d, J* = *7,4 Hz, 2H) ; 2,77 (s, 3H).*
*Analyse spectroscopique de RMN ¹³C (CDCl₃, 75,5 MHz, δ en ppm) : 158,2 (s)* ; *133,0 (s)* ; *133,0 (s) ; 130,6 (s) ; 130,4 (d)* ; *129,3 (s) ; 128,0 (d) ; 127,7 (d) ; 123,2 (d)* ; *118,4 (d)* ; *101,9 (d) ; 67,9 (t) ; 55,5 (q) ; 37,4 (q) ; 33,3 (t).*

### Stade D : N-[2-(7-méthoxynaphtalén-1-yl)éthyl]acétamide

Dans un ballon, sont introduits le produit du Stade C précédent (356 mg ; 1,271 mmol), l'acétonitrile (7 mL) et une solution aqueuse d'ammoniaque à 35 % (5 mL). Le ballon est placé dans un bain chauffé à 110 °C et la solution est agitée pendant 3 heures. La solution est diluée avec de l'acétate d'éthyle et lavée avec une solution aqueuse de soude 2 M, de l'eau et de la saumure, puis séchée sur sulfate de sodium et filtrée. Après évaporation du solvant, le brut est dissous dans l'anhydride acétique (2 mL) en présence d'acétate de sodium (500 mg). Au bout d'une heure d'agitation, de l'eau et de l'acétate d'éthyle sont additionnés et, après 15 minutes d'agitation, la phase organique est lavée deux fois avec une solution aqueuse de soude 2 M, lavée avec de l'eau et de la saumure, séchée sur sulfate de sodium et filtrée. Le solvant est évaporé et le brut obtenu est ensuite purifié par chromatographie sur colonne de gel de silice (éluant : acétate d'éthyle) pour donner le produit du titre (230 mg ; 74 %).
*Analyse spectroscopique de RMN ¹H (CD₃OD, 300,13 MHz, δ en ppm): 8,21 (ls, 1H) ; 7,74 (d, J = 8,9 Hz, 1H)* ; *7,65 (d, J = 8,0 Hz, 1H) ; 7,52 (d, J = 2,5 Hz, 1H) ; 7,31-7,2 (m, 2H) ; 7,11 (dd, J = 8,9 et 2,5 Hz, 1H) ; 3,96 (s, 3H) ; 3,52-3,44 (m, 2H) ; 3,23-3,18 (m, 2H) ; 1,94 (s, 3H).*
*Analyse spectroscopique de RMN ¹³C (CD₃OD, 75,5 MHz, δ en ppm) : 173,4 (s) ; 159,4 (s)* ; *135,1 (s)* ; *134,6 (s) ; 131,2 (d) ; 130,8 (s) ; 128,2 (d) ; 127,9 (d)* ; *124,2 (d) ; 119,3 (d)* ; *103,2 (d) ; 55,9 (q) ; 41,4 (t) ; 34,2 (t) ; 22,6 (q).*

### EXEMPLE 8: N-[2-(7-méthoxynaphtalén-1-yl)éthyl]acétamide

### Stade A : 2-(2-hydroxy-7-méthoxynaphtalén-1-yl)acétamide

Dans un flacon scellé, sont introduits une solution méthanolique d'ammoniac 7 N (10 mL ; 70 mmol) et le produit obtenu lors du Stade A de l'Exemple 3 (1,5 g ; 7 mmol). Au bout de 18 heures d'agitation à 100 °C, le solvant est évaporé pour donner le produit du titre brut (1,58 g ; 98 %) utilisé directement sans autre purification supplémentaire.
*Analyse spectroscopique de RMN ¹H (DMSO-d₆, 300,13 MHz, δ en ppm) : 9,87 (ls, 1H); 7,68 (d, J* = *9,0 Hz, 1H) ; 7,59 (d, J* = *8,7 Hz, 1H) ; 7,36 (ls, 1H) ; 7,18 (d, J* = *2,4 Hz, 1H) ; 7,01 (d, J* = *8,7 Hz, 1H) ; 7,01 (ls, 1H) ; 6,94 (dd, J* = *9,0 et 2,4 Hz, 1H) ; 3,84 (s, 3H) ; 3,79 (s, 2H).*

### Stade B : acétate de 1-[2-(acétylamino)éthyl]-7-méthoxynaphtalén-2-yle

A une suspension du produit du Stade A précédent (1,205 g ; 5,216 mmol) dans le tétrahydrofurane (25 mL), est ajouté l'hydrure de lithium et d'aluminium (396 mg ; 10,432 mmol). Après agitation au reflux pendant 8 heures, le mélange est refroidi à 0 °C et de l'eau (40 mL) puis de l'acide citrique (10 g) sont additionnés. Au bout de 14 heures d'agitation, le milieu est neutralisé en utilisant une solution aqueuse saturée de NaHCO₃ et le produit est extrait trois fois par de l'acétate d'éthyle. Les phases organiques sont rassemblées, lavées à l'eau et avec de la saumure, puis séchées sur sulfate de sodium et filtrées. Après évaporation, le brut (802 mg) est dissous dans l'anhydride acétique (3 mL) en présence d'acétate de sodium (500 mg). Le mélange est agité pendant 16 heures pour être ensuite versé dans une solution aqueuse diluée de NaHCO₃. Le produit est extrait trois fois par l'acétate d'éthyle et les phases organiques sont rassemblées, lavées à l'eau et avec de la saumure, séchées sur sulfate de sodium et filtrées. Après évaporation des solvants, le brut est purifié par chromatographie sur colonne de gel de silice (éluant : acétate d'éthyle / méthanol, gradient de 100/0 à 95/5) pour donner le produit du titre sous forme d'un solide blanc (337 mg ; 21 %).
*Point de fusion : 149-151 °C.*
*Analyse spectroscopique de RMN ¹H (CDCl₃, 300, 13 MHz, δ en ppm) : 7,73 (d, J* = *8,9 Hz, 1H)* ; *7,68 (d, J* = *8,8 Hz, 1H) ; 7,42 (d, J* = *2,2 Hz, 1H) ; 7,14 (dd, J* = *8,8 et 2,2 Hz, 1H) ; 7,01 (d, J* = *8,9 Hz, 1H) ; 5,75 (ls, 1H) ; 3,97 (s, 1H) ; 3,52 (m, 2H) ; 3,19 (t, J* = *6,7 Hz, 2H)* ; *2,39 (s, 3H) ; 1,9 (s, 3H).*
*Analyse spectroscopique de RMN ¹³C (CDCl₃,* 75,5 *MHz, δ en ppm) : 170,7 (s)* ; *170,6 (s) ; 158,6 (s)* ; *147,5 (s)* ; *134,3 (s) ; 130,3 (d) ; 128,2 (d) ; 127,5 (s)* ; *124,1 (s)* ; *119,0 (d) ; 118,2 (d)* ; *102,9 (d)* ; *55,6 (q) ; 39,1 (t) ; 25,9 (t) ; 23,4 (q) ; 21,1 (q).*

### Stade C : 4-méthylbenzènesulfonate de 1-[2-(acétylamino)éthyl]-7-méthoxy naphialén-2-yle

A une solution de soude (61 mg ; 1,528 mmol) dans l'éthanol absolu (10 mL), est ajouté le produit du Stade B précédent (230 mg ; 0,764 mmol). Après agitation pendant une heure, le solvant est évaporé. Le résidu est repris dans un mélange acétate d'éthyle / solution aqueuse diluée d'acide chlorhydrique. Après séparation, la phase organique est lavée à l'eau et avec de la saumure, puis séchées sur sulfate de sodium et filtrées. Après évaporation du solvant, le brut est dissous dans le dichlorométhane (4 mL) en présence de triéthylamine (150 µL ; 2,47 mmol) et de chlorure de tosyle (174 mg ; 0,917 mmol). Au bout de 16 heures d'agitation à température ambiante, le solvant est évaporé. Le résidu est repris dans l'acétate d'éthyle et l'eau. Après séparation, la phase organique est lavée à l'eau et avec de la saumure, séchées sur sulfate de sodium et filtrées. Après évaporation du solvant, le brut est purifié par chromatographie sur colonne de gel de silice (éluant : acétate d'éthyle) pour donner le produit du titre (261 mg ; 83 %).
*Analyse spectroscopique de RMN ¹H_(CDCl₃, 300,13 MHz, δ en ppm) : 7,81 (d, J* = 8,2 *Hz, 2H) ; 7,68 (d, J* = *8,9 Hz, 1H) ; 7,62 (d, J* = *2,4 Hz, 1H) ; 7,55 (d, J* = *8,9 Hz, 1H) ; 7,36 (d, J* = *8,2 Hz, 2H) ; 7,14 (dd, J* = *8,9 et 2,4 Hz, 1H) ; 6,89 (d, 8,9 Hz, 1H) ; 5,97 (m, 1H) ; 4,01 (s, 3H) ; 3,53-3,46 (m, 2H) ; 3,22-3,17 (m, 2H) ; 2,46 (s, 3H) ; 1,95 (s, 3H).*
*Analyse spectroscopique de RMN ¹³C (CDCl₃, 75,5 MHz, δ en ppm) : 170,9 (s)* ; *158,9 (s)* ; *146,4 (s) ; 145,7 (s) ; 134,6 (s) ; 133,3 (s) ; 130,1 (2 x d) ; 130,0 (d)* ; *128,5 (2 x d) ; 128,2 (d) ; 127,7 (s) ; 126,2 (s) ; 119,2 (d) ; 117,8 (d) ; 103,3 (d)* ; *55,8 (q) ; 39,4 (t) ; 26,4 (t)* ; *23,4 (q) ; 21,9 (q).*

### Stade D : N-[2-(7-méthoxynaphtalén-1-yl)éthyl]acétamide

Le produit du titre est obtenu selon le procédé décrit dans le Stade D de l'Exemple 2 en utilisant le produit du Stade C précédent comme réactif de départ.
*Analyse spectroscopique de RMN ¹H (CD₃OD, 300,13 MHz, δ en ppm) : 8,21 (ls, 1H) ; 7,74 (d, J* = *8,9 Hz, 1H) ; 7,65 (d, J* = *8,0 Hz, 1H) ; 7,52 (d, J* = *2,5 Hz, 1H) ; 7,31-7,2 (m, 2H) ; 7,11 (dd, J* = *8,9 et 2,5 Hz, 1H) ; 3,96 (s, 3H) ; 3,52-3,44 (m, 2H) ; 3,23-3,18 (m, 2H) ; 1,94 (s, 3H).*
*Analyse spectroscopique de RMN ¹³C (CD₃OD, 75,5 MHz, δ en ppm) : 173,4 (s) ; 159,4 (s) ; 135,1 (s)* ; *134,6 (s) ; 131,2 (d) ; 130,8 (s) ; 128,2 (d)* ; *127,9 (d) ; 124,2 (d)* ; *119,3 (d) ; 103,2 (d) ; 55,9 (q) ; 41,4 (t) ; 34,2 (t) ; 22,6 (q).*

### EXEMPLE 9 : N-[2-(7-méthoxynaphtalén-1-yl)éthyl]acétamide

### Stade A : 4-méthylbenzènesulfonate de 1-(2-amino-2-oxoéthyl)-7-méthoxy naphtalén-2-yle

A une solution du produit obtenu lors du Stade A de l'Exemple 8 (376 mg ; 1,628 mmol) dans le diméthylformamide (3 mL), est ajouté à 0 °C l'hydrure de sodium (60 % dans l'huile minérale ; 78 mg ; 1,953 mmol). Au bout de 30 minutes d'agitation, le chlorure de tosyle (341 mg ; 1,79 mmol) est introduit en une portion. Après 2 heures d'agitation, le milieu réactionnel est dilué dans l'acétate d'éthyle puis lavé deux fois à l'eau et deux fois à la saumure. La phase organique est séchée sur sulfate de sodium et filtrée. Après évaporation des solvants, le brut est purifié par chromatographie sur colonne de gel de silice (éluant : acétate d'éthyle / dichlorométhane 30/70) pour donner le produit du titre sous forme d'un solide blanc (260 mg ; 40 %).
*Analyse spectroscopique de RMN ¹H (CDCl₃, 300,13 MHz, δ en ppm) : 7,89 (d, J = 7,9 Hz, 2H) ; 7,72 (d, J = 9,0 Hz, 1H) ; 7,66 (d, J = 9,0 Hz, 1H) ; 7,4 (d, J = 7,9 Hz, 2H) ; 7,37 (d, J* = *2,4 Hz, 1H) ; 7,17 (dd, J* = *9,0 et 2,4 Hz, 1H) ; 7,01 (d, J* = *9,0 Hz, 1H) ; 6,02 (ls, 1H) ; 5,48 (ls, 1H) ; 3,93 (s, 2H) ;* 3,91 *(s, 3H) ; 2,50 (s, 3H).*

### Stade B : 2-(7-méthoxynaphtalén-1-yl)acétamide

Dans un flacon scellé, sont introduits le produit obtenu lors du Stade A précédent (50 mg ; 0,13 mmol), le chlorure de nickel hexahydrate (31 mg ; 0,13 mmol), le dichlorométhane (1,3 mL) et le méthanol (1,3 mL). On fait ensuite bullé de l'argon pendant 5 minutes dans la solution, puis le borohydrure de sodium (88 mg ; 2,6 mmol) est ajouté avec précaution par petites portions. Après agitation pendant 1 heure sous argon et à température ambiante, de l'eau est ajoutée. Au bout de 15 minutes d'agitation, le mélange est filtré sur Célite puis lavé au dichlorométhane et à l'acétate d'éthyle. La fraction organique est séchée sur sulfate de sodium puis filtrée. Après évaporation des solvants, le brut est purifié par chromatographie sur colonne de gel de silice (éluant : acétate d'éthyle) pour donner le produit du titre (8 mg ; 29 %).
*Analyse spectroscopique de RMN ¹H (DMSO-d₆, 300,13 MHz, δ en ppm) : 7,84 (d, J = 9,0 Hz, 1H) ; 7,73 (d, J* = *8,0 Hz, 1H) ; 7,62 (ls, 1H) ; 7,41-7,36 (m, 2H) ; 7,28 (dd, J* = *8,0 et 7,1 Hz, 1H) ; 7,18 (dd, J* = *9,0 et 2,4 Hz, 1H) ; 7,02 (ls, 1H) ; 3,88 (s, 3H) ;* 3,82 *(s, 2H).*
*Analyse spectroscopique de RMN ¹³C (DMSO-d₆, 75,5 MHz, δ en ppm) : 172,3 (s) ; 157,3 (s)* ; *133,2 (s)* ; 131,8 *(s)* ; *130,0 (d)* ; *128,7 (s) ; 128,4 (d) ; 126,7 (d)* ; *123,1 (d) ; 117,7 (d)* ; *103,4 (d)* ; *55,2 (q) ; 40,2 (t).*

### Stade C : (7-méthoxynaphtalén-1-yl)acétonitrile

Le produit du titre est obtenu selon le protocole décrit dans le Stade F de la Préparation 1 du brevet EP 0 447 285 en utilisant le produit du Stade B précédent comme réactif de départ.
*Point de fusion : 86-87 °C.*
*Analyse spectroscopique de RMN ¹H (CDCl₃, 300,13 MHz, δ en ppm) : 7,78 (d, J* = *8,9 Hz, 1H) ; 7,77 (d, J* = *7,8 Hz, 1H) ; 7,52 (d, J = 7,1 Hz, 1H) ; 7,32 (dd, J* = *7,8 et 7,1 Hz, 1H) ; 7,21 (dd, J* = *8,9 et 2,4 Hz, 1H) ; 7,03 (d, J* = *2,4 Hz, 1H) ; 4,0 (s, 2H) ; 3,94 (s, 3H).*
*Analyse spectroscopique de RMN ¹³C (CDCl₃, 75,5 MHz, δ en ppm) : 158,5 (s) ; 132,0 (s) ; 130,6 (d) ; 129,1 (s) ; 128,8 (d) ; 127,1 (d) ; 124,4 (s) ; 123,2 (d) ; 118,8 (d) ; 117,7 (s) ; 101,3 (d) ; 55,4 (q) ; 21,9 (t).*

### Stade D : N-[2-(7-méthoxynaphtalén-1-yl)éthyl]acétamide

Le produit du titre est obtenu selon le procédé décrit dans le Stade E de l'Exemple 1 en utilisant le produit du Stade C précédent.
*Point de fusion : 108 °C.*
*Analyse spectroscopique de RMN ¹H (CD₃OD, 300,13 MHz, δ en ppm) : 8,21 (ls, 1H) ; 7,74 (d, J* = *8,9 Hz, 1H) ; 7,65 (d, J* = *8,0 Hz, 1H) ; 7,52 (d, J* = *2,5 Hz, 1H) ; 7,31-7,2 (m, 2H) ; 7,11 (dd, J* = *8,9 et 2,5 Hz, 1H) ; 3,96 (s, 3H) ; 3,52-3,44 (m, 2H) ; 3,23-3,18 (m, 2H) ; 1,94 (s, 3H).*
*Analyse spectroscopique de RMN ¹³C (CD₃OD, 75,5 MHz, δ en ppm) : 173,4 (s) ; 159,4 (s)* ; *135,1 (s) ; 134,6 (s) ; 131,2 (d) ; 130,8 (s) ; 128,2 (d) ; 127,9 (d) ; 124,2 (d) ; 119,3 (d)* ; *103,2 (d) ; 55,9 (q) ; 41,4 (t) ; 34,2 (t) ; 22,6 (q).*

### EXEMPLE 10 : N-[2-(7-méthoxynaphtalén-1-yl)éthyl)acétamide

### Stade A : 2-méthoxy-7-(prop-2-én-1-yloxy)naphtalène

A une solution de 7-méthoxy-naphtalén-2-ol (5,865 g ; 33,71 mmol) et de carbonate de potassium (13,96 g; 101,12 mmol) dans l'acétone (33 mL), est additionné le bromure d'allyle (4,4 mL ; 50,56 mmol). Après agitation à 65 °C pendant 16 heures, le mélange est refroidi à température ambiante et de l'eau est ajoutée (60 mL). Après agitation pendant 3 heures, le produit est extrait trois fois par de l'acétate d'éthyle. Les fractions organiques sont rassemblées, lavées deux à l'eau puis avec de la saumure, séchées sur sulfate de sodium et filtrées. L'évaporation des solvants fournit un brut (7,963 g) utilisé directement dans l'étape suivante sans plus de purification.
*Analyse spectroscopique de RMN ¹H (CDCl₃, 300,13 MHz, δ en ppm) : 7,68 (d, J* = *8,9 Hz, 1H) ; 7,67 (d, J* = *8,9 Hz, 1H) ; 7,08-6,99 (m, 3H) ; 6,13 (m, 1H) ; 5,48 (m, 1H); 5,34 (m, 1H)* ; *4,65 (m, 1H) ; 3,92 (s, 3H).*

### Stade B : 7-méthoxy-1-(prop-2-én-1-yl)naphtalén-2-ol

Dans un ballon, le produit du Stade A précédent (7,963 g ; 33,71 mmol) est mis dans un bain chauffé à 200 °C pendant 2,5 heures. Après refroidissement, le brut est utilisé directement dans l'étape suivante sans plus de purification.
*Analyse spectroscopique de RMN ¹H (CDCl₃, 300,13 MHz, δ en ppm) : 7,69 (d, J* = *8,9 Hz, 1H)* ; *7,6 (d, J* = *8, 7 Hz, 1H) ; 7,2 (d, J* = *2,4 Hz, 1H) ; 7,03 (dd, J* = *8,9 et 2,4 Hz, 1H) ; 6,95 (d, J* = *8, 7 Hz, 1H) ; 6,08 (m, 1H) ;* 5,28 *(s, 1H) ; 5,14 (m, 1H) ; 5,1 (m, 1H) ; 3,93 (s, 3H)* ; *3,8 (m, 1H).*

### Stade C : 4-méthylbenzènesulfonate de 7-méthoxy-1-(prop-2-én-1-yl)naphtalén-2-yle

A une solution du produit du Stade B précédent (2,5 g; 11,68 mmol) dans le dichlorométhane (22 mL), sont ajoutés la triéthylamine (1,95 mL ; 14,02 mmol) et le chlorure de tosyle (2,23 g ; 11,68 mmol). Après agitation pendant 16 heures à température ambiante, le solvant est évaporé et le résidu est repris dans l'acétate d'éthyle et l'eau. Après séparation, la phase organique est lavée à l'eau et avec de la saumure, séchée sur sulfate de sodium et filtrée. Après évaporation du solvant, le brut est purifié par chromatographie sur colonne de gel de silice (éluant : acétate d'éthyle / éther de pétrole, gradient de 10 / 90 à 20 / 80) pour donner le produit du titre (3,79 g ; 88 %).
*Analyse spectroscopique de RMN ¹H (CDCl₃, 300,13 MHz, δ en ppm) : 7,8 (d, J* = *8,3 Hz, 2H) ; 7,71 (d, J* = *8,9 Hz, 1H)* ; *7,6 (d, J* = *8,9 Hz, 1H) ; 7,33 (d, J* = *8,3 Hz, 2H)* ; *7,24 (d, J* = *2,5 Hz, 1H) ; 7,14 (dd, J* = *8,9 et 2,5 Hz) ; 7,1 (d, J* = *8,9 Hz, 1H) ; 5, 79 (m, 1H)* ; *5,02-4,95 (m, 2H) ; 3,88 (s, 3H) ; 3,68 (d, J = 5,9 Hz, 2H) ; 2,46 (s, 3H).*
*Analyse spectroscopique de RMN ¹³C (CDCl₃, 75,5 MHz, δ en ppm) : 158,3 (s) ; 146,1 (s) ; 145,5 (s) ; 135,5 (d) ; 134,4 (s) ; 133,3 (s) ; 130,1 (d)* ; *130,0 (2 x d)* ; *128,5 (2 x d) ; 128,0 (d) ; 127,7 (s) ; 126,6 (s)* ; *118,5 (d) ; 118,4 (d) ; 116,1 (t) ; 103,9 (d) ; 55,4 (q) ; 30,5 (t)* ; *21,8 (q).*

### Stade D : 4-méthylbenzènesulfonate de 1-(2,3-dihydroxypropyl)-7-méthoxy naphtalén-2-yle

A une solution du produit du Stade C précédent (3,786 g ; 10,29 mmol) dans l'acétone (40 mL) et l'eau déionisée (10 mL), sont ajoutés la 4-méthylmorpholine *N*-oxyde hydratée (1,666 g ; 12,35 mmol) et le tétroxyde d'osmium (4 % dans l'eau ; 653 µL ; 0,01 mmol). Après agitation à température ambiante pendant 20 heures, le thiosulfate de sodium pentahydrate est additionné (1 g). Au bout d'une nouvelle heure d'agitation, les solvants sont évaporés. Le résidu est repris dans l'acétate d'éthyle et la phase organique est lavée avec de la saumure, séchée sur sulfate de sodium et filtrée. Après évaporation du solvant, le brut est purifié par chromatographie sur colonne de gel de silice (éluant : acétate d'éthyle) pour donner le produit du titre sous forme d'un solide blanc (3,617 g ; 87 %).
*Analyse spectroscopique de RMN ¹H (CDCl₃, 300,13 MHz, δ en ppm) : 7,81 (d, J = 8,3 Hz, 2H) ; 7,69 (d, J = 9,0 Hz, 1H) ; 7,58 (d, J = 9,0 Hz, 1H) ; 7,33 (d, J* = *8,3 Hz, 2H) ; 7,13 (dd, J = 9,0 et 2,4, 1H) ; 7,02 (d, J = 9,0 Hz, 1H) ; 4,01 (m, 1H) ; 3,88 (s, 3H) ; 3,63-3,47 (m, 2H) ; 3,11 (d, J = 7,0 Hz, 2H) ; 2,9 (ls, 1H) ; 2,62 (ls, 1H) ; 2,44 (s, 3H).*
*Analyse spectroscopique de RMN ¹³C (CDCl₃, 75,5 MHz, δ en ppm) : 158,6 (s) ; 146,8 (s)* ; *145,8 (s) ; 134,6 (s) ; 133,1 (s) ; 130,2 (d) ; 130,1 (2 x d) ; 128,4 (2 x d) ; 128,3 (d) ; 127,7 (s) ; 125,4 (s) ; 118,6 (d) ; 118,0 (d) ; 103,6 (d) ; 72,0 (d) ; 65,8 (t) ; 55,5 (q) ; 30,1 (t) ; 21,8 (q).*

### Stade E : 4-méthylbenzènesulfonate de 7-méthoxy-1-(2-oxoéthyl)naphtalén-2-yle

A une solution du produit du Stade D précédent (1,23 g ; 3,06 mmol) dans le tétrahydrofurane (12 mL) et l'eau (3 mL), sont ajoutés le periodate de sodium (785 mg ; 3,671 mmol) et une solution aqueuse de HCl 2 M (1,8 mL). Au bout de 30 minutes, le mélange est neutralisé avec une solution aqueuse diluée de NaHCO₃ et le produit est ensuite extrait par de l'acétate d'éthyle. Les fractions organiques sont rassemblées, séchées sur sulfate de sodium et filtrées. L'évaporation des solvants fournit le produit du titre sous forme d'un solide blanc propre (1,13 g ; 100 %), directement utilisé dans l'étape suivante. *Point de fusion : 101-103 °C.*
*Analyse spectroscopique de RMN ¹H (CDCl₃, 300,13 MHz, δ en ppm) : 9,43 (t, J* = *2,6 Hz, 1H) ; 7,78 (d, J* = *8,3 Hz, 2H)* ; *7,74 (d, J* = *8,9 Hz, 1H) ; 7,68 (d, J* = *8,9 Hz, 1H) ; 7,35 (d, J* = *8,3 Hz, 2H) ;* 7,17 *(dd, J* = *8,9 et 2,4 Hz, 1H) ; 7,04 (d, J* = *8,9 Hz, 1H) ; 6,97 (d, J* = *2,4 Hz, 1H) ; 3,98 (d, J* = *2,6 Hz, 2H) ; 3,88 (s, 3H) ; 2,46 (s, 3H).*
*Analyse spectroscopique de RMN ¹³C (CDCl₃, 75,5 MHz, δ en ppm) : 199,2 (d) ; 159,1 (s) ; 146,8 (s) ; 146,0 (s) ; 134,7 (s) ; 132,7 (s) ; 130,4 (d) ; 130,2 (2 x d) ; 129,4 (d) ; 128,6 (2 x d)* ; *127,6 (s) ; 120,1 (s) ; 119,1 (d) ; 118,6 (d) ; 102,9 (d) ;* 55,5 *(q) ; 41,7 (t) ; 21,9 (q).*

### Stade F : 2-(7-méthoxynaphtalén-1-yl)éthanol

A une solution du produit du Stade E précédent (950 mg ; 2,567 mmol) dans le méthanol (13 mL), sont ajoutés sous argon le chlorure de nickel (366 mg ; 2,824 mmol) et le borohydrure de sodium (1,3 g ; 38,2 mmol) par petites portions. Au bout d'une heure d'agitation, le mélange est hydrolysé avec une solution aqueuse de HCl 2 M (80 mL) et est ensuite agité pendant 30 minutes en présence d'acétate d'éthyle. La solution hétérogène est filtrée sur Célite et lavée avec de l'acétate d'éthyle. Les deux phases sont séparées et la fraction organique est lavée avec de la saumure, séchée sur sulfate de sodium et filtrée. Après évaporation du solvant, le brut est purifié par chromatographie sur colonne de gel de silice (éluant : acétate d'éthyle / éther de pétrole 40/60) pour donner le produit du titre sous forme d'un solide blanc (442 mg ; 85 %).
*Analyse spectroscopique de RMN ¹H (CDCl_{3,} 300,13 MHz, δ en ppm)* : *7,72 (d, J* = *9,1 Hz, 1H) ; 7,63 (d, J* = *8,1 Hz, 1H) ; 7,3-7,21 (m, 3H) ; 7,13 (dd, J* = *9,1 et 2,6 Hz, 1H) ; 3,93 (t, J* = *6,7 Hz, 2H) ; 3,88 (s, 3H)* ; *3,24 (d, J* = *6,7 Hz, 2H) ; 1,99 (ls, 1H).*
*Analyse spectroscopique de RMN ¹³C (CDCl_{3,} 75,5 MHz, δ en ppm): 157,8 (s)* ; *133,2 (s) ; 133,0 (s) ; 130,4 (d) ; 129,4 (s)* ; *127,7 (d) ; 127,1 (d) ; 123,3 (d) ; 118,1 (d) ; 102,5 (d)* ; *62,7 (t) ; 55,4 (q) ; 36,4 (t).*
*Spectrométrie de masse (ESI ; m*/*z(%)) : 202(29) [M]*⁺*^{•} ; 171(100).*

### Stade G : méthanesulfonate de 2-(7-méthoxynaphtalén-1-yl)éthyle

Le produit du titre est obtenu selon le procédé décrit dans le Stade C de l'Exemple 7 en utilisant le produit du Stade F précédent comme réactif de départ.
*Analyse spectroscopique de RMN ¹H (CDCl_{3,} 300,13 MHz, δ en ppm)* : *7,72 (d, J* = *9,1 Hz, 1H)* ; *7,66 (d, J* = *8,9 Hz, 1H) ; 7,31-7,21 (m, 3H) ; 7,13 (dd, J* = *9,1 et 2,5 Hz, 1H) ; 4,47 (t, J* = *7,4 Hz, 2H) ; 3,92 (s, 3H) ; 3,45 (d, J* = *7,4 Hz, 2H) ; 2,77 (s, 3H).*
*Analyse spectroscopique de RMN ¹³C (CDCl₃, 75,5 MHz, δ en ppm) : 158,2 (s) ; 133,0 (s)* ; *133,0 (s)* ; *130,6 (s) ; 130,4 (d) ; 129,3 (s)* ; *128,0 (d) ; 127,7 (d) ; 123,2 (d) ; 118,4 (d)* ; *101,9 (d) ; 67,9 (t) ; 55,5 (q) ; 37,4 (q) ; 33,3 (t).*

### Stade H : N-[2-(7-méthoxynaphtalén-1yl)éthyl]acétamide

Le produit du titre est obtenu selon le procédé décrit dans le Stade D de l'Exemple 7 en utilisant le produit du Stade G précédent comme réactif de départ. *Analyse spectroscopique de RMN ¹H (CD₃OD, 300,13 MHz, δ ppm) : 8,21 (ls, 1H) ; 7,74 (d, J* = *8,9 Hz, 1H) ; 7,65 (d, J* = *8,0 Hz, 1H) ; 7,52 (d, J* = *2,5 Hz, 1H) ; 7,31-7,2 (m, 2H)* ; *7,11 (dd, J* = *8,9 et 2,5 Hz, 1H) ; 3,96 (s, 3H) ; 3,52-3,44 (m, 2H) ; 3,23-3,18 (m*, *2H)* ; *1,94 (s, 3H).*
*Analyse spectroscopique de RMN ¹³C (CD₃OD, 75,5 MHz, δ en ppm): 173,4 (s) ; 159,4 (s) ; 135,1 (s) ; 134,6 (s) ; 131,2 (d) ; 130,8 (s) ; 128,2 (d)* ; *127,9 (d) ; 124,2 (d) ; 119,3 (d) ; 103,2 (d) ; 55,9 (q) ; 41,4 (t) ; 34,2 (t) ; 22,6 (q).*

### EXEMPLE 11 : N-[2-(7-méthoxynaphtalén-1-yl)éthyl]acétamide

### Stade A : 3-(7-méthoxynaphialén-1-yl)propane-1,2-diol

Dans un flacon scellé, sont introduits sous argon le produit obtenu lors du Stade D de l'Exemple 10 (44 mg ; 0,11 mmol), le chlorure de nickel (16 mg; 0,12 mmol) et le méthanol (1 mL). Le borohydrure de sodium (74 mg ; 2,19 mmol) est ensuite ajouté avec précaution par petites portions. Au bout de 2 heures d'agitation, de l'eau (1 mL) puis le peroxyde d'hydrogène (35 % dans l'eau; 1 mL) sont additionnés et le mélange est agité pendant 2 heures. De la saumure est ensuite ajoutée et la phase aqueuse est extraite trois fois par de l'acétate d'éthyle. Les phases organiques sont rassemblées, séchées sur sulfate de sodium et filtrées. L'évaporation des solvants donne un produit propre (22 mg ; 86 %) sans besoin de purification supplémentaire.
*Analyse spectroscopique de RMN ¹H (CDCl_{3,} 300,13 MHz, δ en ppm) : 7,75 (d, J* = *9,1 Hz, 1H) ; 7,68 (d, J* = *8,1 Hz, 1H) ; 7,32-7,23 (m, 3H) ; 7,16 (dd, J* = *9,1 et 2,4 Hz, 1H) ; 4,11 (m, 1H) ; 3,92 (s, 3H)* ; *3,72-3,55 (m, 2H) ; 3,25-3,1 (m, 2H) ; 2,54 (ls, 2H, OH).*

### Stade B : (7-méthoxynaphtalén-1-yl)acétcildéhyde

Le produit du titre est obtenu selon le procédé décrit dans le Stade E de l'Exemple 10 en utilisant le produit du Stade A précédent à la place du 4-méthylbenzènesulfonate de 1-(2,3-dihydroxypropyl)-7-méthoxynaphtalén-2-yle.
*Analyse spectroscopique de RMN ¹H (CDCl₃, 300, 13 MHz, δ en ppm) : 9,74 (t, J* = *2,6 Hz, 1H); 7,74-7,84 (m, 2H) ; 7,38 (d, J* = *6,0 Hz, 1H)* ; *7,32 (t, J* = *7,5 Hz, 1H) ; 7,19 (dd, J* = *9,0 Hz et 2,4 Hz, 1H) ; 7,1 (d, J* = *2,4 Hz, 1H) ; 4,05 (d, J* = *2,6 Hz, 2H) ; 3,92 (s, 3H).*
*Analyse spectroscopique de RMN ¹³C (CDCl₃, 300, 13 MHz, δ en ppm)* : *199,85 ; 158,29,-133,54; 130,44; 129,32; 129,07 ; 128,23 ; 126,83 ; 123,36; 118,63; 102,12; 55,36; 48,83.*

### Stade C : 2-(7-méthoxynaphtalén-1-yl)éthanol

A une solution du produit du Stade B précédent (20 mg ; 0,1 mmol) dans l'éthanol (1 mL), est ajouté le borohydrure de sodium (38 mg ; 1 mmol). Au bout de 2 heures d'agitation à température ambiante, le mélange est hydrolysé avec une solution aqueuse de HCl 2 M (2 mL) et est ensuite agité pendant 30 minutes en présence d'acétate d'éthyle (1 mL). La solution est extraite 4 fois avec de l'acétate d'éthyle. Les fractions organiques sont regroupées, séchées sur sulfate de sodium et filtrées. Après évaporation du solvant, le brut est purifié par chromatographie sur colonne de gel de silice (éluant : acétate d'éthyle / éther de pétrole 20 / 80) pour donner le produit du titre sous forme d'un solide blanc (19,8 mg ; 98 %).
*Analyse spectroscopique de RMN ¹H (CDCl*₃, *300,13 MHz, δ en ppm) : 7,72 (d, J* = *9,1 Hz, 1H)* ; *7,63 (d, J* = *8,1 Hz, 1H) ; 7,3-7,21 (m, 3H)* ; *7,13 (dd, J* = *9,1 et 2,6 Hz, 1H) ; 3,93 (t, J* = *6,7 Hz, 2H) ; 3,88 (s, 3H) ; 3,24 (d, J* = *6,7 Hz, 2H) ; 1,99 (ls, 1H).*
*Analyse spectroscopique de RMN ¹³C (CDCl₃, 75,5 MHz, δ en ppm) : 157,8 (s)* ; *133,2 (s) ; 133,0 (s) ; 130,4 (d) ; 129,4 (s) ; 127,7 (d) ; 127,1 (d) ; 123,3 (d) ; 118,1 (d) ; 102,5 (d)* ; *62,7 (t) ; 55,4 (q) ; 36,4 (t).*
*Spectrométrie de masse (ESI; m*/*z (%)) : 202(29) [M]*⁺*^{•} ; 171(100).*

### Stade D méthanesulfonate de 2-(7-méthoxynaphtalén-1-yl)éthyle

Le produit du titre est obtenu selon le procédé décrit dans le Stade C de l'Exemple 7 en utilisant le produit du Stade C précédent comme réactif de départ.
*Analyse spectroscopique de RMN ¹H (CDCl₃, 300, 13 MHz, δ en ppm)*: *7,72 (d, J* = *9,1 Hz, 1H)* ; *7,66 (d, J* = *8,9 Hz, 1H) ; 7,31-7,21 (m, 3H) ; 7,13 (dd, J* = *9,1 et 2,5 Hz, 1H) ; 4,47 (t, J* = *7,4 Hz, 2H) ; 3,92 (s, 3H) ; 3,45 (d, J* = *7,4 Hz, 2H) ; 2,77 (s, 3H).*
*Analyse spectroscopique de RMN ¹³C (CDCl₃, 75,5 MHz, δ en ppm) : 158,2 (s)* ; *133,0 (s) ; 133,0 (s) ; 130,6 (s)* ; *130,4 (d) ; 129,3 (s) ; 128,0 (d) ; 127,7 (d) ; 123,2 (d)* ; *118,4 (d) ; 101,9 (d) ; 67,9 (t)* ; *55,5 (q) ; 37,4 (q) ; 33,3 (t).*

### Stade E: N-[2-(7-méthoxynaphtalén-1-yl)éthyl]acétamide

Le produit du titre est obtenu selon le procédé décrit dans le Stade D de l'Exemple 7 en utilisant le produit du Stade D précédent comme réactif de départ.
*Analyse spectroscopique de RMN ¹H (CD₃OD, 300,13 MHz, δ en ppm) : 8,21 (ls, 1H) ; 7,74 (d, J* = *8,9 Hz, 1H); 7,65 (d, J* = *8,0 Hz, 1H) ; 7,52 (d, J* = *2,5 Hz, 1H) ; 7,31-7,2 (m, 2H) ; 7,11 (dd, J* = *8,9 et 2,5 Hz, 1H) ; 3,96 (s, 3H) ; 3,52-3,44 (m, 2H) ; 3,23-3,18* (*m*, *2H) ; 1,94 (s, 3H).*
*Analyse spectroscopique de RMN ¹³C (CD₃OD, 75,5 MHz, δ en ppm) : 173,4 (s) ; 159,4 (s) ; 135,1 (s) ; 134,6 (s) ; 131,2 (d) ; 130,8 (s)* ; 128,2 *(d)* ; *127,9 (d) ; 124,2 (d) ; 119,3 (d) ; 103,2 (d) ; 55,9 (q) ; 41,4 (t) ; 34,2 (t) ; 22,6 (q).*

## Revendications

1. Procédé de synthèse industriel du composé de formule (I) : 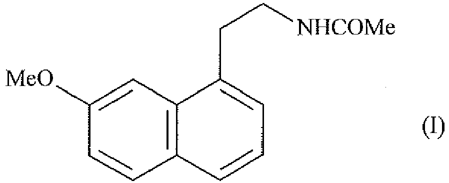 **caractérisé en ce que** l'on met en réaction le 7-méthoxy-naphtalén-2-ol de formule (II) : 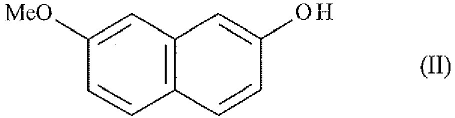 sur lequel on introduit sur la position 1 du composé de formule (II) le groupement -CH₂-X dans lequel X représente un groupement -N(CH₃)₂, -CO-N(CH₂-Ph)₂, -CH₂-OH, -CH=CH₂ ou -CO-NH₂,
pour conduire au composé de formule (III) : 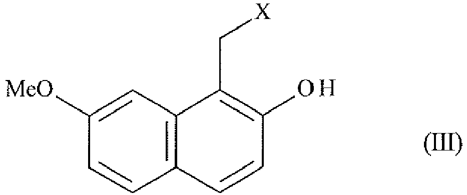 dans laquelle X représente un groupement -N(CH₃)₂, -CO-N(CH₂-Ph)₂, -CH₂-OH, -CH=CH₂ ou -CO-NH₂ ;
composé de formule (III) qui est soumis à une réaction de sulfonylation sur l'alcool aromatique et dont le substituant X est modifié, avant ou après l'étape de sulfonylation de l'alcool aromatique, au moyen de réactions chimiques classiques pour conduire au composé de formule (IV) : 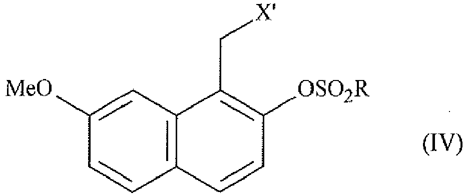 dans laquelle X' représente un groupement -CN, -CO-NH₂, -CH₂-OH, -CHO, -CH₂-N(CH₂-Ph)₂, -CH₂-NH-CO-CH₃, -CH(OH)-CH₂-OH ou (2,5-dioxopyrrolidin-1-yl)méthyle et R représente un groupement -CH₃, -(CH₂)₂-CH₃, -CF₃ ou toluyle ;
composé de formule (IV) qui subit une réaction de désoxygénation en présence d'un métal de transition et d'un agent réducteur pour conduire :
- soit, lorsque X' représente le groupement -CH₂-NH-CO-CH₃, directement au composé de formule (I) que l'on isole sous forme d'un solide ;
- soit au composé de formule (V) : 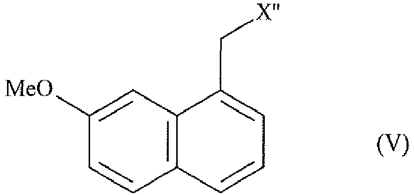 dans laquelle X" représente un groupement -CN, -CH₂-N(CH₂-Ph)₂, -CH₂-OH, -CH(OH)-CH₂-OH, -CO-NH₂ ou (2,5-dioxopyrrolidin-1-yl)méthyle ;
composé de formule (V) qui est soumis à des réactions chimiques classiques pour conduire au composé de formule (I) que l'on isole sous forme d'un solide.

2. Procédé de synthèse industriel du composé de formule (I) selon la revendication 1 **caractérisé en ce que** la transformation du composé de formule (II) en composé de formule (III) est réalisée par action du formaldéhyde et de la diméthylamine pour conduire au composé de formule (III) dans laquelle X représente -N(CH₃)₂.

3. Procédé de synthèse industriel du composé de formule (I) selon la revendication 1 **caractérisé en ce que** la transformation du composé de formule (II) en composé de formule (III) est réalisée par action du glyoxal suivie de l'action d'un agent réducteur pour conduire au composé de formule (III) dans laquelle X représente -CH₂-OH.

4. Procédé de synthèse industriel du composé de formule (I) selon la revendication 1 **caractérisé en ce que** la transformation du composé de formule (II) en composé de formule (III) est réalisée par action du glyoxal suivie de l'action du composé de formule NHR'R' dans laquelle R' représente H ou un groupement -CH₂-Ph, pour conduire au composé de formule (III) dans laquelle X représente -CO-NH₂ ou -CO-N(CH₂-Ph)₂.

5. Procédé de synthèse industriel du composé de formule (I) selon la revendication 1 **caractérisé en ce que** la transformation du composé de formule (II) en composé de formule (III) est réalisée par action du bromure d'allyle suivie d'un réarrangement thermique pour conduire au composé de formule (III) dans laquelle X représente -CH=CH₂.

6. Procédé de synthèse industriel du composé de formule (I) selon la revendication 1 **caractérisé en ce que**, lors de la transformation du composé de formule (III) en composé de formule (IV), l'étape de sulfonylation est réalisée grâce à l'action d'un chlorure de sulfonyle, d'un anhydride sulfonique ou d'un sulfonimide.

7. Procédé de synthèse industriel du composé de formule (I) selon la revendication 1 **caractérisé en ce que** la transformation du composé de formule (III) en composé de formule (IV) consiste en une étape de sulfonylation de l'alcool aromatique suivie de la modification du groupement X au moyen de réactions chimiques classiques, X étant tel que défini dans la formule (III).

8. Procédé de synthèse industriel du composé de formule (I) selon la revendication 1 **caractérisé en ce que** la transformation du composé de formule (III) en composé de formule (IV) consiste en la modification du groupement X au moyen de réactions chimiques classiques suivie d'une étape de sulfonylation de l'alcool aromatique, X étant tel que défini dans la formule (III).

9. Procédé de synthèse industriel du composé de formule (I) selon la revendication 1 **caractérisé en ce que** la transformation du composé de formule (IV) en composé de formule (V) est réalisée en présence de nickel et d'un hydrure.

10. Procédé de synthèse industriel du composé de formule (I) selon la revendication 1 **caractérisé en ce que** la transformation du composé de formule (IV) en composé de formule (V) est réalisée en présence de palladium et de dihydrogène.

11. Procédé de synthèse industriel du composé de formule (I) selon la revendication 1 **caractérisé en ce que** la transformation du composé de formule (IV) en composé de formule (V) est réalisée en présence de palladium et d'un métal alcalino-terreux.

12. Procédé de synthèse industriel du composé de formule (I) selon la revendication 1 **caractérisé en ce que** la transformation du composé de formule (IV) dans laquelle X' représente le groupement -CH₂-NH-CO-CH₃ en composé de formule (I) est réalisée en présence de nickel et d'un hydrure.

13. Procédé de synthèse industriel du composé de formule (I) selon la revendication 1 **caractérisé en ce que** la transformation du composé de formule (IV) dans laquelle X' représente le groupement -CH₂-NH-CO-CH₃ en composé de formule (I) est réalisée en présence de palladium et de dihydrogène.

14. Procédé de synthèse industriel du composé de formule (I) selon la revendication 1 **caractérisé en ce que** la transformation du composé de formule (IV) dans laquelle X' représente le groupement -CH₂-NH-CO-CH₃ en composé de formule (I) est réalisée en présence de palladium et d'un métal alcalino-terreux.

15. Composé de formule (III) selon la revendication 1 utile en tant qu'intermédiaire de synthèse de l'agomélatine de formule (I).

16. Composé de formule (III) selon la revendication 15 choisi parmi les composés suivants :
- 1-[(diméthylamino)méthyl]-7-méthoxynaphtalén-2-ol ;
- *N,N-*dibenzyl-2-(2-hydroxy-7-méthoxynaphtalén-1-yl)acétamide ;
- 1-(2-hydroxyéthyl)-7-méthoxynaphtalén-2-ol ;
- 2-(2-hydroxy-7-méthoxynaphtalén-1-yl)acétamide;
- 7-méthoxy-1-(prop-2-én-1-yl)naphtalén-2-ol.

17. Utilisation du composé de formule (III) selon la revendication 15 ou 16 dans la synthèse de l'agomélatine de formule (I).

18. Composé de formule (IV) selon la revendication 1 utile en tant qu'intermédiaire de synthèse de l'agomélatine de formule (I).

19. Composé de formule (IV) selon la revendication 18 choisi parmi les composés suivants :
- trifluorométhanesulfonate de 1-(cyanométhyl)-7-méthoxynaphtalén-2-yle ;
- 4-méthylbenzènesulfonate de 1-[2-(acétylamino)éthyl]-7-méthoxynaphtalén-2-yle ;
- trifluorométhanesulfonate de 1-[2-(dibenzylamino)éthyl]-7-méthoxynaphtalén-2-yle;
- propane-1-sulfonate de 1-[2-(acétylamino)éthyl]-7-méthoxynaphtalén-2-yle ;
- propane-1-sulfonate de 1-[2-(2,5-dioxopyrrolidin-1-yl)éthyl]-7-méthoxynaphtalén-2-yle ;
- 4-méthylbenzènesulfonate de 1-(2-hydroxyéthyl)-7-méthoxynaphtalén-2-yle ;
- 4-méthylbenzènesulfonate de 1-(2-amino-2-oxoéthyl)-7-méthoxynaphtalén-2-yle ;
- 4-méthylbenzènesulfonate de 7-méthoxy-1-(2-oxoéthyl)naphtalén-2-yle ;
- 4-méthylbenzènesulfonate de 1-(2,3-dihydroxypropyl)-7-méthoxynaphtalén-2-yle.

20. Utilisation du composé de formule (IV) selon la revendication 18 ou 19 dans la synthèse de l'agomélatine de formule (I).

21. Composé de formule (V) selon la revendication 1 choisi parmi les composés suivants :
- 1-[2-(7-méthoxynaphtalén-1-yl)éthyl]pyrrolidine-2,5-dione ;
- 3-(7-méthoxynaphtalén-1-yl)propane-1,2-diol ;
utiles en tant qu'intermédiaires de synthèse de l'agomélatine de formule (I).

22. Utilisation du composé de formule (V) selon la revendication 21 dans la synthèse de l'agomélatine de formule (I).

23. (2-hydroxy-7-méthoxynaphtalén-1-yl)acétonitrile, 4-méthylbenzènesulfonate de 7-méthoxy-1-(2-{[(4-méthylphényl)sulfonyl]oxy} éthyl)naphtalén-2-yle, propane-1-sulfonate de 7-méthoxy-1-{2-[(propylsulfonyl)oxy]éthyl}naphtalén-2-yle et acétate de 1-[2-(acétylamino)éthyl]-7-méthoxynaphtalén-2-yle utiles en tant qu'intermédiaires de synthèse de l'agomélatine de formule (I).

24. Utilisation du (2-hydroxy-7-méthoxynaphtalén-1-yl)acétonitrile, du 4-méthylbenzènesulfonate de 7-méthoxy-1-(2-{[(4-méthylphényl)sulfonyl]oxy}éthyl) naphtalén-2-yle, du propane-1-sulfonate de 7-méthoxy-1-{2-[(propylsulfonyl)oxy] éthyl}naphtalén-2-yle et de l'acétate de 1-[2-(acétylamino)éthyl]-7-méthoxynaphtalén-2-yle selon la revendication 23 dans la synthèse de l'agomélatine de formule (I).

25. Composé de formule (VI) : 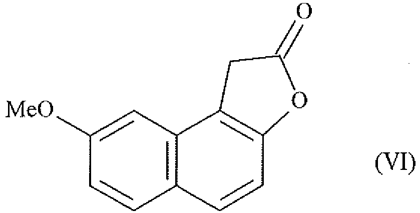 utile en tant qu'intermédiaire de synthèse de l'agomélatine de formule (I).

26. Utilisation du composé de formule (VI) selon la revendication 25 dans la synthèse de l'agomélatine de formule (I).

27. Utilisation du composé de formule (II) selon la revendication 1 dans la synthèse de l'agomélatine de formule (I).

28. Procédé de synthèse de l'agomélatine selon la revendication 1 à partir du composé de formule (III) **caractérisé en ce que** le composé de formule (III) est obtenu par le procédé de synthèse selon l'une des revendications 1 à 5.

29. Procédé de synthèse de l'agomélatine selon la revendication 1 à partir du composé de formule (IV) **caractérisé en ce que** le composé de formule (IV) est obtenu par le procédé de synthèse selon l'une des revendications 1 à 8.

## Patentansprüche

1. Verfahren zur industriellen Synthese der Verbindung der Formel (I): 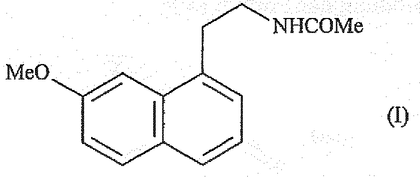 **dadurch gekennzeichnet, dass** man 7-Methoxy-naphthalin-2-ol der Formel (II): 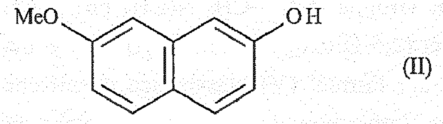 umsetzt zur Einführung der Gruppe -CH₂-X in die Stellung 1 der Verbindung der Formel (II), , worin X eine Gruppe -N(CH₃)₂, -CO-N(CH₂-Ph)₂, -CH₂-OH, -CH=CH₂ oder -CO-NH₂ bedeutet,
zur Bildung der Verbindung der Formel (III): 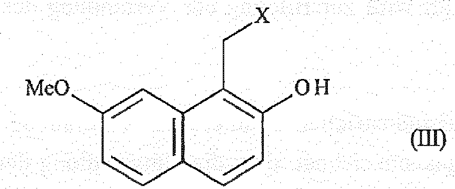 in der X eine Gruppe -N(CH₃)₂, -CO-N(CH₂-Ph)₂, -CH₂-OH, -CH=CH₂ oder -CO-NH₂ bedeutet;
welche Verbindung der Formel (III) einer Sulfonylierungsreaktion am aromatischen Alkohol unterworfen wird und deren Substituent X vor oder nach der Stufe der Sulfonylierung des aromatischen Alkohols mit Hilfe von klassischen chemischen Reaktionen modifiziert wird zur Bildung der Verbindung der Formel (IV): 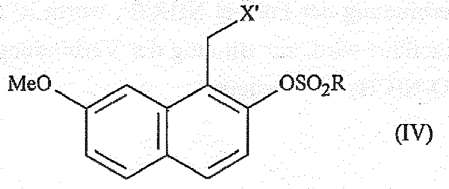 in der X' eine Gruppe -CN, -CO-NH₂, -CH₂-OH, -CHO, -CH₂-N(CH₂-Ph)₂, -CH₂-NH-CO-CH₃, -CH(OH)-CH₂-OH oder (2,5-Dioxopyrrolidin-1-yl)-methyl und R eine Gruppe -CH₃, -(CH₂)₂-CH₃, -CF₃ oder eine Toluylgruppe bedeuten,
welche Verbindung der Formel (IV) einer Desoxygenierungsreaktion in Gegenwart eines Übergangsmetalls und eines Reduktionsmittels unterworfen wird zur Bildung:
- entweder, wenn X' die Gruppe -CH₂-NH-CO-CH₃ bedeutet, direkt der Verbindung der Formel (I), die man in Form eines Feststoffs isoliert;
- oder der Verbindung der Formel (V): 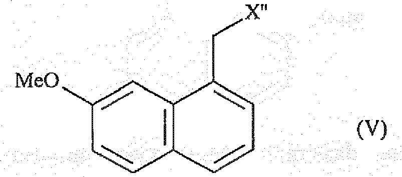 in der X" eine Gruppe -CN, -CH₂-N(CH₂-Ph)₂, -CH₂-OH, -CH(OH)-CH₂-OH, -CO-NH₂ oder (2,5-Dioxopyrrolidin-1-yl)-methyl bedeutet;
welche Verbindung der Formel (V) klassischen chemischen Reaktionen unterworfen wird zur Bildung der Verbindung der Formel (I), welche man in Form eines Feststoffs isoliert.

2. Verfahren zur industriellen Synthese der Verbindung der Formel (I) nach Anspruch 1, **dadurch gekennzeichnet, dass** die Umwandlung der Verbindung der Formel (II) in die Verbindung der Formel (III) durch Einwirkung von Formaldehyd und Dimethylamin durchgeführt wird zur Bildung der Verbindung der Formel (III), in der X - N(CH₃)₂ bedeutet.

3. Verfahren zur industriellen Synthese der Verbindung der Formel (I) nach Anspruch 1, **dadurch gekennzeichnet, dass** die Umwandlung der Verbindung der Formel (II) in die Verbindung der Formel (III) durch Einwirkung von Glyoxal, gefolgt von der Einwirkung eines Reduktionsmittels durchgeführt wird zur Bildung der Verbindung der Formel (III), in der X -CH₂-OH bedeutet.

4. Verfahren zur industriellen Synthese der Verbindung der Formel (I) nach Anspruch 1, **dadurch gekennzeichnet, dass** die Umwandlung der Verbindung der Formel (II) in die Verbindung der Formel (III) durch Einwirkung von Glyoxal, gefolgt von der Einwirkung der Verbindung der Formel NHR'R', worin R' H oder eine Gruppe -CH₂-Ph bedeutet, durchgeführt wird, zur Bildung der Verbindung der Formel (III), in der X -CO-NH₂ oder -CO-N(CH₂-Ph)₂ bedeutet.

5. Verfahren zur industriellen Synthese der Verbindung der Formel (I) nach Anspruch 1, **dadurch gekennzeichnet, dass** die Umwandlung der Verbindung der Formel (II) in die Verbindung der Formel (III) durch Einwirkung von Allylbromid, gefolgt von einer thermischen Umlagerung durchgeführt wird zur Bildung der Verbindung der Formel (III), in der X -CH=CH₂ bedeutet.

6. Verfahren zur industriellen Synthese der Verbindung der Formel (I) nach Anspruch 1, **dadurch gekennzeichnet, dass** bei der Umwandlung der Verbindung der Formel (III) in die Verbindung der Formel (IV) die Stufe der Sulfonylierung durch die Einwirkung eines Sulfonylchlorids, eines Sulfonsäureanhydrids oder eines Sulfonimids erfolgt.

7. Verfahren zur industriellen Synthese der Verbindung der Formel (I) nach Anspruch 7, **dadurch gekennzeichnet, dass** die Umwandlung der Verbindung der Formel (III) in die Verbindung der Formel (IV) in einer Stufe der Sulfonylierung des aromatischen Alkohols, gefolgt von der Modifizierung der Gruppe X mit Hilfe klassischer chemischer Reaktionen erfolgt, worin X die bezüglich der Formel (III) angegebenen Bedeutungen besitzt.

8. Verfahren zur industriellen Synthese der Verbindung der Formel (I) nach Anspruch 1, **dadurch gekennzeichnet, dass** die Umwandlung der Verbindung der Formel (III) in die Verbindung der Formel (IV) in der Modifizierung der Gruppe X mit Hilfe von klassischen chemischen Reaktionen besteht, gefolgt von einer Stufe der Sulfonylierung des aromatischen Alkohols, worin X die bezüglich der Formel (III) angegebenen Bedeutungen besitzt.

9. Verfahren zur industriellen Synthese der Verbindung der Formel (I) nach Anspruch 1, **dadurch gekennzeichnet, dass** die Umwandlung der Verbindung der Formel (IV) in die Verbindung der Formel (V) in Gegenwart von Nickel und eines Hydrids durchgeführt wird.

10. Verfahren zur industriellen Synthese der Verbindung der Formel (I) nach Anspruch 1, **dadurch gekennzeichnet, dass** die Umwandlung der Verbindung der Formel (IV) in die Verbindung der Formel (V) in Gegenwart von Palladium und Wasserstoff durchgeführt wird.

11. Verfahren zur industriellen Synthese der Verbindung der Formel (I) nach Anspruch 1, **dadurch gekennzeichnet, dass** die Umwandlung der Verbindung der Formel (IV) in die Verbindung der Formel (V) in Gegenwart von Palladium und eines Erdalkalimetalls durchgeführt wird.

12. Verfahren zur industriellen Synthese der Verbindung der Formel (I) nach Anspruch 1, **dadurch gekennzeichnet, dass** die Umwandlung der Verbindung der Formel (IV), in der X' die Gruppe -CH₂-NH-CO-CH₃ bedeutet, in die Verbindung der Formel (I) in Gegenwart von Nickel und eines Hydrids durchgeführt wird.

13. Verfahren zur industriellen Synthese der Verbindung der Formel (I) nach Anspruch 1, **dadurch gekennzeichnet, dass** die Umwandlung der Verbindung der Formel (IV), worin X' die Gruppe -CH₂-NH-CO-CH₃ bedeutet, in die Verbindung der Formel (I) in Gegenwart von Palladium und Wasserstoff durchgeführt wird.

14. Verfahren zur industriellen Synthese der Verbindung der Formel (I) nach Anspruch 1, **dadurch gekennzeichnet, dass** die Umwandlung der Verbindung der Formel (IV), in der X' die Gruppe -CH₂-NH-CO-CH₃ bedeutet, in die Verbindung der Formel (I) in Gegenwart von Palladium und eines Erdalkalimetalls durchgeführt wird.

15. Verbindung der Formel (III) nach Anspruch 1, nützlich als Zwischenprodukt bei der Synthese von Agomelatin der Formel (I).

16. Verbindung der Formel (III) nach den Ansprüchen 15, ausgewählt aus den folgenden Verbindungen:
- 1 -[(Dimethylamino)-methyl]-7-methoxynaphthalin-2-ol;
- *N,N*-Dibenzyl-2-(2-hydroxy- -yl)-acetamid;
- 1-(2-Hydroxyethyl)-7-methoxynaphthalin-2-ol;
- 2-(2-Hydroxy-7-methoxynaphthalin-l-yl)-acetamid;
- 7-Methoxy-1-(prop-2-en-1-yl)-naphthalin-2-ol.

17. Verwendung der Verbindung der Formel (II) nach Anspruch 15 oder 16 bei der Synthese von Agomelatin der Formel (I).

18. Verbindung der Formel (IV) nach Anspruch 1, nützlich als Zwischenprodukt bei der Synthese von Agomelatin der Formel (I).

19. Verbindung der Formel (IV) nach Anspruch 18, ausgewählt aus den folgenden Verbindungen:
- 1-(Cyanomethyl)-7-methoxynaphthalin-2-yl-trifluormethansulfonat;
- 1-[2-(Acetylamino)-ethyl]-7-methoxynaphthalin-2-yl-4-methylbenzolsulfonat;
- 1-[2-(Dibenzylamino)-ethyl]-7-methoxynaphthalin-2-yl-trifluormethansulfonat;
- 1-[2-(Acetylamino)-ethyl]-7-methoxynaphthalin-2-yl-propan-1-sulfonat;
- 1-[2-(2,5-Dioxopyrrolidin-1-yl)-ethyl]-7-methoxynaphthalin-2-yl-propan-1-sulfonat;
- 1-(2-Hydroxyethyl)-7-methoxynaphthalin-2-yl-4-methylbenzolsulfonat;
- 1-(2-Amino-2-oxoethyl)-7-methoxynaphthalin-2-yl-4-methylbenzolsulfonat;
- 7-Methoxy-1-(2-oxoethyl)-naphthalin-2-yl-4-methylbenzolsulfonat;
- 1-(2,3-Dihydroxypropyl)-7-methoxynaphthalin-2-yl-4-methylbenzolsulfonat.

20. Verwendung der Verbindung der Formel (IV) nach Anspruch 18 oder 19 bei der Synthese von Agomelatin der Formel (I).

21. Verbindung der Formel (V) nach Anspruch 1, ausgewählt aus den folgenden Verbindungen:
- 1-[2-(7-Methoxynaphthalin-1-yl)-ethyl]-pyrrolidin-2,5-dion;
- 3-(7-Methoxynaphthalin-1-yl)-propan-1,2-diol;
nützlich als Zwischenprodukte bei der Synthese von Agomelatin der Formel (I).

22. Verwendung der Verbindung der Formel (IV) nach Anspruch 21 bei der Synthese von Agomelatin der Formel (I).

23. (2-Hydroxy-7-methoxynaphthalin-1-yl)-acetonitril, 7-Methoxy-1-(2- {[(4-methylphenyl)-sulfonyl]-oxy}-ethyl)-naphthalin-2-yl-4-methylbenzolsulfonat, 7-Methoxy-1-{2-[(propylsulfonyl)-oxy]-ethyl}-naphthalin-2-yl-propan-1-sulfonat und 1-[2-(Acetylamino)-ethyl]-7-methoxynaphthalin-2-yl-acetat nützlich als Zwischenprodukte bei der Synthese von Agomelatin der Formel (I).

24. Verwendung von (2-Hydroxy-7-methoxynaphthalin-1-yl)-acetonitril, 7-Methoxy-1-(2-{[(4-methylphenyl)-sulfonyl]-oxy}-ethyl)-naphthalin-2-yl-4-methylbenzolsulfonat, 7-Methoxy-1-{2-[(propylsulfonyl)-oxy]-ethyl}-naphthalin-2-yl-propan-1-sulfonat und 1-[2-(Acetylamino)-ethyl]-7-methoxynaphthalin-2-yl-acetat nach Anspruch 23 bei der Synthese von Agomelatin der Formel (I).

25. Verbindung der Formel (VI): 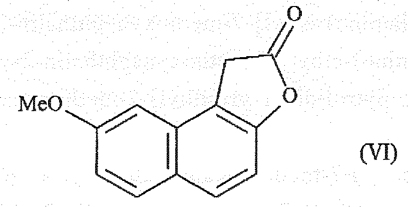 nützlich als Zwischenprodukt bei der Synthese von Agomelatin der Formel (I).

26. Verwendung der Verbindung der Formel (VI) nach Anspruch 25 bei der Synthese von Agomelatin der Formel (I).

27. Verwendung der Verbindung der Formel (II) nach Anspruch 1 bei der Synthese von Agomelatin der Formel (I).

28. Verfahren zur Synthese von Agomelatin nach Anspruch 1, ausgehend von der Verbindung der Formel (III), **dadurch gekennzeichnet, dass** die Verbindung der Formel (III) nach dem Syntheseverfahren gemäß einem der Ansprüche 1 bis 5 erhalten wird.

29. Verfahren zur Synthese von Agomelatin nach Anspruch 1, ausgehend von der Verbindung der Formel (IV), **dadurch gekennzeichnet, dass** die Verbindung der Formel (IV) durch das Syntheseverfahren gemäß einem der Ansprüche 1 bis 8 erhalten wird.

## Claims

1. Process for the industrial synthesis of the compound of formula (I): 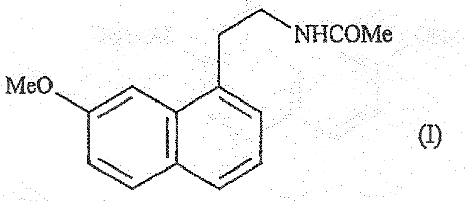 **characterised in that** 7-methoxy-naphthalen-2-ol of formula (II): 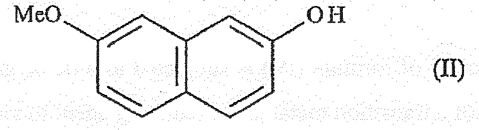 is reacted, wherein there is introduced in position 1 of the compound of formula (II) the group -CH₂X wherein X represents a group -N(CH₃)₂, -CON(CH₂-Ph)₂, -CH₂-OH, -CH=CH₂ or -CO-NH₂,
to yield the compound of formula (III): 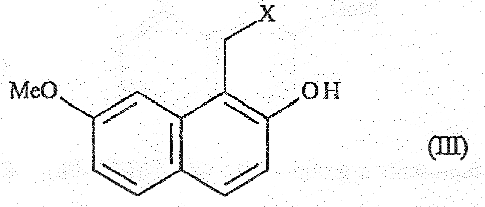 wherein X represents a group -N(CH₃)₂, -CO-N(CH₂-Ph)₂, -CH₂-OH, -CH=CH₂ or -CO-NH₂;
which compound of formula (III) is subjected to a sulfonylation reaction on the aromatic alcohol and the substituent X of which is modified, before or after the step of sulfonylation of the aromatic alcohol, by means of conventional chemical reactions to yield the compound of formula (IV): 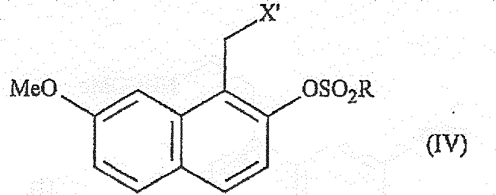 wherein X' represents a group -CN, -CO-NH₂, -CH₂-OH, -CHO, -CH₂-N(CH₂-Ph)₂, -CH₂-NH-CO-CH₃, -CH(OH)-CH₂-OH or (2,5-dioxopyrrolidin-1-yl)methyl and R represents a group -CH₃, -(CH₂)₂-CH₃, -CF₃ or toluyl;
which compound of formula (IV) is subjected to a deoxygenation reaction in the presence of a transition metal and a reducing agent to yield:
- either, when X' represents the group -CH₂-NH-CO-CH₃, the compound of formula (I) directly, which is isolated in the form of a solid;
- or the compound of formula (V): 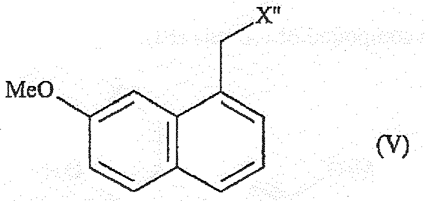
wherein X" represents a group -CN, -CH₂-N(CH₂-Ph)₂, -CH₂OH, -CH(OH)-CH₂-OH, -CO-NH₂ or (2,5-dioxopyrrolidin-1-yl)methyl;
which compound of formula (V) is subjected to conventional chemical reactions to yield the compound of formula (I), which is isolated in the form of a solid.

2. Process for the industrial synthesis of the compound of formula (I) according to claim 1, **characterised in that** the conversion of the compound of formula (II) into the compound of formula (III) is carried out by the action of formaldehyde and dimethylamine to yield the compound of formula (III) wherein X represents -N(CH₃)₂.

3. Process for the industrial synthesis of the compound of formula (I) according to claim 1, **characterised in that** the conversion of the compound of formula (II) into the compound of formula (III) is carried out by the action of glyoxal followed by the action of a reducing agent to yield the compound of formula (III) wherein X represents -CH₂-OH.

4. Process for the industrial synthesis of the compound of formula (I) according to claim 1, **characterised in that** the conversion of the compound of formula (II) into the compound of formula (III) is carried out by the action of glyoxal followed by the action of the compound of the formula NHR'R' wherein R' represents H or a group -CH₂-Ph to yield the compound of formula (III) wherein X represents -CO-NH₂ or -CO-N(CH₂-Ph)₂.

5. Process for the industrial synthesis of the compound of formula (I) according to claim 1, **characterised in that** the conversion of the compound of formula (II) into the compound of formula (III) is carried out by the action of allyl bromide followed by a thermal rearrangement to yield the compound of formula (III) wherein X represents -CH=CH₂.

6. Process for the industrial synthesis of the compound of formula (I) according to claim 1, **characterised in that**, in the conversion of the compound of formula (III) into the compound of formula (IV), the sulfonylation step is carried out by means of the action of a sulfonyl chloride, a sulfonic anhydride or a sulfonimide.

7. Process for the industrial synthesis of the compound of formula (I) according to claim 1, **characterised in that** the conversion of the compound of formula (III) into the compound of formula (IV) consists in a step of sulfonylation of the aromatic alcohol followed by modification of the group X by means of conventional chemical reactions, X being as defined for formula (III).

8. Process for the industrial synthesis of the compound of formula (I) according to claim 1, **characterised in that** the conversion of the compound of formula (III) into the compound of formula (IV) consists in modifying the group X by means of conventional chemical reactions followed by a step of sulfonylation of the aromatic alcohol, X being as defined for formula (III).

9. Process for the industrial synthesis of the compound of formula (I) according to claim 1, **characterised in that** the conversion of the compound of formula (IV) into the compound of formula (V) is carried out in the presence of nickel and a hydride.

10. Process for the industrial synthesis of the compound of formula (I) according to claim 1, **characterised in that** the conversion of the compound of formula (IV) into the compound of formula (V) is carried out in the presence of palladium and dihydrogen.

11. Process for the industrial synthesis of the compound of formula (I) according to claim 1, **characterised in that** the conversion of the compound of formula (IV) into the compound of formula (V) is carried out in the presence of palladium and an alkaline earth metal.

12. Process for the industrial synthesis of the compound of formula (I) according to claim 1, **characterised in that** the conversion of the compound of formula (IV) wherein X' represents the group -CH₂-NH-CO-CH₃ into the compound of formula (I) is carried out in the presence of nickel and a hydride.

13. Process for the industrial synthesis of the compound of formula (I) according to claim 1, **characterised in that** the conversion of the compound of formula (IV) wherein X' represents the group -CH₂-NH-CO-CH₃ into the compound of formula (I) is carried out in the presence of palladium and dihydrogen.

14. Process for the industrial synthesis of the compound of formula (I) according to claim 1, **characterised in that** the conversion of the compound of formula (IV) wherein X' represents the group -CH₂-NH-CO-CH₃ into the compound of formula (I) is carried out in the presence of palladium and an alkaline earth metal.

15. Compound of formula (III) according to claim 1 for use as an intermediate for the synthesis of agomelatine of formula (I).

16. Compound of formula (III) according to claim 15 chosen from the following compounds:
- 1-[(dimethylamino)methyl]-7-methoxynaphthalen-2-ol;
- *N,N*-dibenzyl-2-(2-hydroxy-7-methoxynaphthalen-1-yl)acetamide;
- 1-(2-hydroxyethyl)-7-methoxynaphthalen-2-ol;
- 2-(2-hydroxy-7-methoxynaphthalen-1-yl)acetamide;
- 7-methoxy-1-(prop-2-en-1-yl)naphthalen-2-ol.

17. Use of the compound of formula (III) according to claim 15 or 16 in the synthesis of agomelatine of formula (I).

18. Compound of formula (IV) according to claim 1 for use as an intermediate for the synthesis of agomelatine of formula (I).

19. Compound of formula (IV) according to claim 18 chosen from the following compounds:
- 1-(cyanomethyl)-7-methoxynaphthalen-2-yl trifluoromethanesulfonate;
- 1-[2-(acetylamino)ethyl]-7-methoxynaphthalen-2-yl 4-methylbenzenesulfonate;
- 1-[2-(dibenzylamino)ethyl]-7-methoxynaphthalen-2-yl trifluoromethanesulfonate;
- 1-[2-(acetylamino)ethyl]-7-methoxynaphthalen-2-yl propane-1-sulfonate;
- 1-[2-(2,5-dioxopyrrolidin-1-yl)ethyl]-7-methoxynaphthalen-2-yl propane-1-sulfonate;
- 1-(2-hydroxyethyl)-7-methoxynaphthalen-2-yl 4-methylbenzenesulfonate;
- 1-(2-amino-2-oxoethyl)-7-methoxynaphthalen-2-yl 4-methylbenzenesulfonate;
- 7-methoxy-1-(2-oxoethyl)naphthalen-2-yl 4-methylbenzenesulfonate;
- 1-(2,3-dihydroxypropyl)-7-methoxynaphthalen-2-yl 4-methylbenzenesulfonate.

20. Use of the compound of formula (IV) according to claim 18 or 19 in the synthesis of agomelatine of formula (I).

21. Compound of formula (V) according to claim 1 chosen from the following compounds:
- 1-[2-(7-methoxynaphthalen-1-yl)ethyl]pyrrolidine-2,5-dione;
- 3-(7-methoxynaphthalen-1-yl)propane-1,2-diol;
for use as intermediates for the synthesis of agomelatine of formula (I).

22. Use of the compound of formula (V) according to claim 21 in the synthesis of agomelatine of formula (I).

23. (2-Hydroxy-7-methoxynaphthalen-1-yl)acetonitrile, 7-methoxy-1-(2-{[(4-methylphenyl)sulfonyl] oxy} ethyl)naphthalen-2-yl 4-methylbenzenesulfonate, 7-methoxy-1-{2-[(propylsulfonyl)oxy]ethyl}naphthalen-2-yl propane-1-sulfonate and 1-[2-(acetylamino)ethyl]-7-methoxynaphthalen-2-yl acetate for use as intermediates for the synthesis of agomelatine of formula (I).

24. Use of (2-hydroxy-7-methoxynaphthalen-1-yl)acetonitrile, 7-methoxy-1-(2-{[(4-methylphenyl)sulfonyl]oxy}ethyl)naphthalen-2-yl 4-methylbenzenesulfonate, 7-methoxy-1- {2- [(propylsulfonyl)oxy] ethyl} naphthalen-2-yl propane-1-sulfonate and 1-[2-(acetylamino)ethyl]-7-methoxynaphthalen-2-yl acetate according to claim 23 in the synthesis of agomelatine of formula (I).

25. Compound of formula (VI): 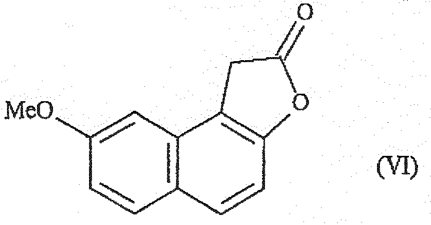 for use as an intermediate for the synthesis of agomelatine of formula (I).

26. Use of the compound of formula (VI) according to claim 25 in the synthesis of agomelatine of formula (I).

27. Use of the compound of formula (II) according to claim 1 in the synthesis of agomelatine of formula (I).

28. Process for the synthesis of agomelatine according to claim 1 starting from the compound of formula (III), **characterised in that** the compound of formula (III) is obtained by the synthesis process according to any one of claims 1 to 5.

29. Process for the synthesis of agomelatine according to claim 1 starting from the compound of formula (IV), **characterised in that** the compound of formula (IV) is obtained by the synthesis process according to any one of claims 1 to 8.
